# EUROPEAN PATENT SPECIFICATION

(11) **EP 0 792 875 B1**
(45) Date of publication and mention of the grant of the patent: **09.05.2001**
(21) Application number: 97301272.7
(22) Date of filing: 26.02.1997
(51) Int. Cl.: C07D 255/02, C07D 257/02, C07D 273/02, C07D 273/08, C07D 273/00, C07D 285/00, C07D 291/02, C07D 413/06, C07D 417/06, C07D 419/06, A61K 31/395

(54) **Cryptophycin derivatives and their use as anti-microtubule agents**
Cryptophycinderivate und ihre Verwendung als antimikrotubuli Mittel
Dérivés de cryptophycine et leur utilisation comme agents antimicrotubules

(30) Priority: 27.02.1996 US 12375; 30.08.1996 US 25081; 27.02.1996 US 12365; 30.08.1996 US 25080; 20.12.1996 US 20633
(43) Date of publication of application: 03.09.1997
(73) Proprietor: ELI LILLY AND COMPANY, Indianapolis, Indiana 46285 (US); UNIVERSITY OF HAWAII, Honolulu, HI 96822 (US); Wayne State University, Detroit, Michigan 48202 (US)
(72) Inventor: Norman, Bryan Hurst, Indianapolis, Indiana 46236 (US); Shih, Chuan, Carmel, Indiana 46033 (US); Kroin, Julian Stanley, Indianapolis, Indiana 46234 (US)
(74) Representative: Hudson, Christopher Mark

(56) References cited:
- WO-A-95/17093

## Description

This invention relates to novel cryptophycin compounds useful as anti-microtubule agents.

Neoplastic diseases, characterized by the proliferation of cells not subject to the normal control of cell growth, are a major cause of death in humans and other mammals. Clinical experience in cancer chemotherapy has demonstrated that new and more effective drugs are desirable to treat these diseases. Such clinical experience has also demonstrated that drugs which disrupt the microtubule system of the cytoskeleton can be effective in inhibiting the proliferation of neoplastic cells.

The microtubule system of eucaryotic cells is a major component of the cytoskeleton and is a dynamic assembly and disassembly; that is heterodimers of tubulin are polymerized and form microtubule. Microtubules play a key role in the regulation of cell architecture, metabolism, and division. The dynamic state of microtubules is critical to their normal function. With respect to cell division, tubulin is polymerized into microtubles that form the mitotic spindle. The microtubules are then depolymerized when the mitotic spindle's use has been fulfilled. Accordingly, agents which disrupt the polymerization or depolymerization of microtubules, and thereby inhibit mitosis, comprise some of the most effective cancer chemotherapeutic agents in clinical use.

Further, such agents having the ability to disrupt the microtubule system can be useful for research purposes.

Certain cryptophycin compounds are known in the literature U.S. Patent Nos. 4,946,835, 4,845,085, 4,845,086, and 4,868,208; however, compounds having greater metabolic stability and longer duration of action are desired for most pharmaceutical uses. Applicants have now discovered novel compounds providing such desired greater metabolic stability as well as the ability to disrupt the microtubule system. Such compounds can be prepared using total synthetic methods and are therefore well suited for development as pharmaceutically useful agents.

WO 95/17093 discloses cryptophycin derivatives useful to inhibit proliferation of hyperproliferative cells with drug resistant phenotypes and to treat neoplasia.

The presently claimed invention provides novel cryptophycin compounds of Formula I 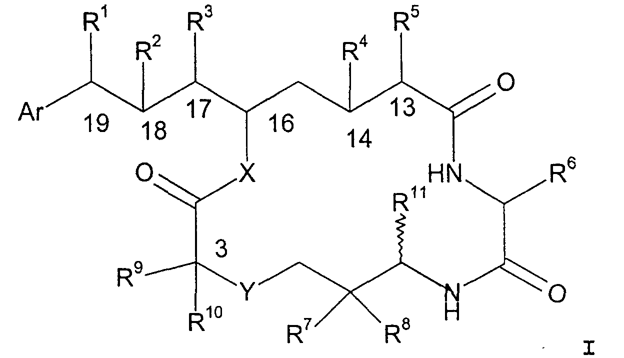 wherein
Ar is selected from the group consisting of- phenyl and a simple substituted aromatic group; R¹ is selected from the group consisting of halogen, SH, amino, monoalkylamino, dialkylamino, trialkylammonium, sulfate, or phosphate;
R² is OH or SH; or
R¹ and R² may be taken together with C₁₈ and C₁₉ to form an epoxide ring, an aziridine ring, an episulfide ring, a sulfate ring, or monoalkylphosphate ring; or
R¹ and R² may be taken together to form a second bond between C₁₈ and C₁₉;
R³ is a lower alkyl group;
R⁴ is H;
R⁵ is H;
R⁴ and R⁵ may be taken together to form a second bond between C₁₃ and C₁₄;
R⁶ is selected from the group consisting of benzyl, hydroxybenzyl, halohydroxybenzyl, dihalohydroxybenzyl and substituted benzyl wherein one substituent is a halogen atom and one substituent is an OR¹² group wherein R¹² is lower alkyl;
R⁷ is H or a lower alkyl group;
R⁸ is H or a lower alkyl group;
R⁷ and R⁸ may optionally be taken together to form a cyclopropyl ring;
R⁹ is selected from the group consisting of H, a lower alkyl group, (C₁-C₃) alkylaryl, and aryl;
R¹⁰ is selected from the group consisting of H, a lower alkyl group, (C₁-C₃) alkylaryl, and aryl;
R¹¹ is selected from the group consisting of simple alkyl; phenyl, substituted phenyl, benzyl, and substituted benzyl; X is O, NH or alkylamino;
Y is C, O, NH, S, SO, SO₂ or alkylamino; or
a pharmaceutically acceptable salt or solvate thereof.

The presently claimed invention provides novel cryptophycin compounds of Formula III 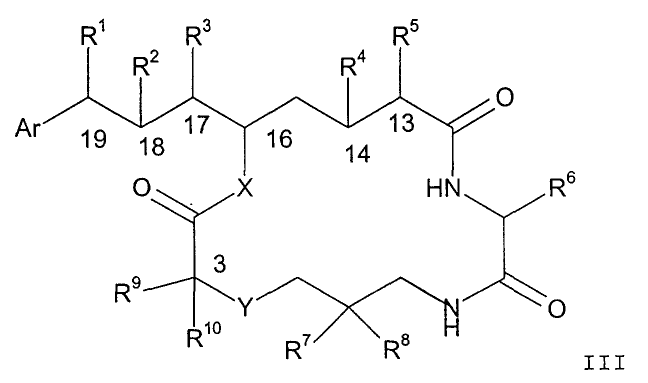 wherein
Ar is selected from the group consisting of phenyl and a simple substituted aromatic group;
R¹ is selected from the group consisting of halogen, SH, amino, monoalkylamino, dialkylamino, trialkylammonium, sulfate and phosphate;
R² is OH or SH; or
R¹ and R² may be taken together with C₁₈ and C₁₉ to form an epoxide ring, an aziridine ring, an episulfide ring, a sulfate ring, or monoalkylphosphate ring; or
R¹ and R² may be taken together to form a second bond between C₁₈ and C₁₉;
R³ is a lower alkyl group;
R⁴ is H;
R⁵ is H;
R⁴ and R⁵ may be taken together to form a second bond between C₁₃ and C₁₄;
R⁶ is selected from the group consisting of benzyl, hydroxybenzyl, halohydroxybenzyl, dihalohydroxybenzyl and substituted benzyl wherein one substituent is a halogen atom and one substituent is an OR¹² group wherein R¹² is lower alkyl;
R⁷ is H or a lower alkyl group;
R⁸ is H or a lower alkyl group;
R⁷ and R⁸ may optionally be taken together to form a cyclopropyl ring;
R⁹ is selected from the group consisting of H, a lower alkyl group, (C₁-C₃) alkylaryl, and aryl;
R¹⁰ is selected from the group consisting of H, a lower alkyl group, (C₁-C₃) alkylaryl, and aryl;
X is O, NH or alkylamino;
Y is C, O, NH, S, SO, SO₂ or alkylamino; or
a pharmaceutically acceptable salt or solvate thereof.

The present invention provides pharmaceutical formulations, compounds for use in a method for disrupting a microtubulin system using an effective amount of a compound of Formula I or Formula III, compounds for use in a method for inhibiting the proliferation of mammalian cells comprising administering an effective amount of a compound of Formula I or Formula III, and compounds for use in a method for treating neoplasia in a mammal comprising administering an effective amount of a compound of Formula I or Formula III.

As used herein, the term "simple alkyl" shall refer to C₁-C₇ alkyl wherein the alkyl may be saturated, unsaturated, branched, or straight chain. Examples include methyl, ethyl, n-propyl, iso-propyl, n-butyl, propenyl, sec-butyl, n-pentyl, isobutyl, tert-butyl, sec-butyl, methylated butyl groups, pentyl, tert pentyl, sec-pentyl and methylated pentyl groups.

As used herein, the term "substituted phenyl" shall refer to a phenyl group with from one to three substituents which may be independently selected from the group consisting of simple alkyl, Cl, Br, F, and I.

As used herein, the term "substituted benzyl" shall refer to a benzyl group with from one to three substitutents which may be independently selected from the group consisting of simple alkyl, Cl, Br, F, and I.

As used herein "lower alkyl group" means an alkyl group of one to five carbons and includes linear and nonlinear hydrocarbon chains, including for example methyl, ethyl, propyl, isopropyl, butyl, isobutyl, tert-butyl, sec-butyl, methylated butyl groups, pentyl, tert pentyl, sec-pentyl, and methylated pentyl groups. As used herein "allylically substituted alkene" means any alkene having from one to seven carbon atoms which contains an alkyl substitution on it.

As used herein "epoxide ring" means a three-membered ring whose backbone consists of two carbons and an oxygen atom. As used herein, "aziridine ring" means a three-membered ring whose backbone consists of two carbon atoms and a nitrogen atom. As used herein "sulfide ring" means a three-membered ring whose backbone consists of two carbon atoms and a sulfur atom. As used herein "episulfide ring" means a three-membered ring whose backbone consists of two carbon and a sulfur atom. As used herein "sulfate group" means a five membered ring consisting of a carbon-carbon-oxygen-sulfur-oxygen backbone with two additional oxygen atoms connected to the sulfur atom. As used herein, "monalkylphosphate ring" means a five membered ring consisting of a carbon-carbon-oxygen-phosphorous-oxygen backbone with two additional oxygen atoms, one of which bears a lower alkyl group, connected to the phosphorous atom.

As used herein "simple substituted aromatic group" refers to a phenyl group substituted with a single group selected from the group consisting of halogen and lower alkyl group.

As used herein, the term " aryl" means phenyl or naphthyl.

The term " C₁-C₃ alkylaryl" represents an (C₁-C₃) alkylaryl substituent wherein the alkyl group is linear, such as benzyl, phenethyl, 3-phenylpropyl, or phenyl-t-butyl; or branched. The alkylaryl moeity is attached to the parent nucleus via the alkyl group.

As used herein, " alkylamino" has its common meaning. Thus, the phrase refers to N-R^{R'} wherein R^{R'} is C₁-C₃ alkyl. When the alkylamino group is contained within the ring as when " X" and/or " Y" is alkylamino then such alkylamino group in the ring can be represented by the group:

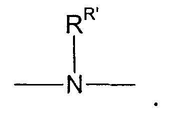

As used herein, "halogen" refers to those members of the group on the periodic table historically known as halogens. Methods of halogenation include
the addition of hydrogen halides, substitution at high temperature, photohalogenation, etc., and such methods are known to the skilled artisan.

As used herein, the term "crypto A-B-OCH₂CCl₃" or "cryptophycin A-Btrichloroethyl ester" shall mean a group of the formula:

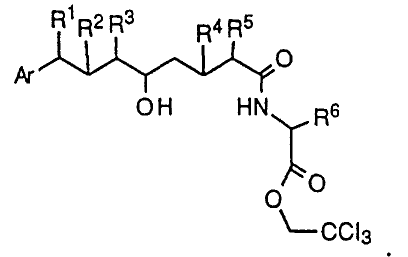

As used herein, the term "mammal" shall refer to the Mammalia class of higher vertebrates. The term "mammal" includes a human. The term "treating" as used herein includes prophylaxis of the named condition or amelioration or elimination of the condition once it has been established. The cryptophycin compounds claimed herein can be useful for vetrinary health purposes as well as for the treatment of a human patient.

Some preferred characteristics of this invention are set forth in the following tabular form wherein the features may be independently selected to provide preferred embodiments of this invention.
A) R⁸ is ethyl, propyl, isopropyl, butyl, isobutyl or isopentyl;
B) R⁷ is ethyl, propyl, isopropyl, butyl, isobutyl, pentyl, or isopentyl;
C) R⁷ is H, R⁸ is methyl, R³ is methyl, and X and Y are not both O;
D) R³ is ethyl, propyl, isopropyl, butyl, isobutyl, pentyl or isopentyl;
E) R⁹ is methyl, ethyl, propyl, butyl, isobutyl, pentyl, or isopentyl;
F) R¹⁰ is methyl, ethyl, propyl, butyl, isobutyl, pentyl, or isopentyl;
G) a cryptophycin compound wherein at least one of the groups selected from the group consisting of C-3, C-6, C-7, C-10, C-16, C-17, and C-18 has *R* stereochemistry (numbering as set forth in claim 1 *infra*.);
H) a cryptophycin compound wherein at least one of the groups selected from the group consisting of C-3, C-6, C-7, C-10, C-16, C-17, and C-18 has *S* stereochemistry (numbering as set forth in claim 1 *infra*.);
I) Ar is phenyl with a substituent selected from the group consisting of hydrogen, halogen, and simple alkyl;
J) a compound wherein the C-7 substituent is *R* configuration;
K) a compound wherein the C-7 substituent is *S* configuration;
L) R⁷, R⁸ are each hydrogen;
M) R⁷ and R⁸ are each selected from hydrogen or OH;
N) R¹¹ is simple alkyl;
O) R is selected from the group consisting of methyl, ethyl, n-propyl, and phenyl;
P) R¹ and R² form an epoxide ring;
Q) both X and Y are O;
R) R⁴ and R⁵ form a double bond;
S) R⁶ is substituted benzyl wherein one substituent is a halogen and one is an OR¹² group wherein R¹² is lower alkyl;
T) a compound of Formula I is used for disruption of a microtubulin system;
U) a compound of Formula I is used as an anti-neoplastic agent;
V) a compound of Formula III is used for the treatment of cancer in a mammal;
W) a compound wherein Y is selected from the group consisting of O, NH, S, SO and SO₂;
X) a compound wherein Y is C, R⁷, R⁸, R⁹, and R¹⁰ are each hydrogen; and R¹ and R² form an epoxide;
Y) Y is O or S;
Z) Y is selected from S, SO, and SO₂; and
Z1) R¹⁰ is hydrogen.

Examples of some preferred compounds of this invention include 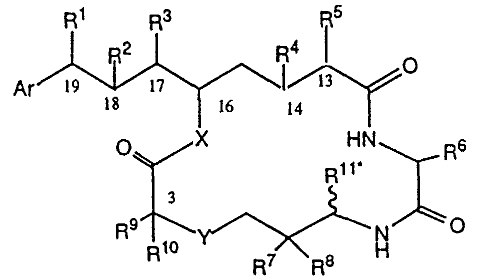 wherein Ar is phenyl, R¹ and R² taken together form a three membered epoxide ring, R³ is methyl, R⁴ and R⁵ are taken together to form a double bond, R⁶ is a chloro-methoxybenzyl, R¹⁰ is hydrogen, and the remaining variables are as illustrated in the following table:

| **X** | **Y** | **R**^{**9**} | **R**^{**7**} | **R**^{**8**} | **R**^{**11***} |
|---|---|---|---|---|---|
| O | O | isobutyl | hydrogen | hydrogen | hydrogen |
| O | O | isobutyl | hydrogen | hydrogen | methyl |
| O | O | isobutyl | hydrogen | hydrogen | benzyl |
| O | O | isobutyl | hydrogen | hydrogen | phenyl |
| O | O | isobutyl | hydrogen | hydrogen | p-chloro- |
| O | O | isobutyl | hydrogen | hydrogen | phenyl ethyl |
| O | O | isobutyl | hydrogen | hydrogen | m-methylbenzyl |
| O | O | isobutyl | hydrogen | hydrogen | p-methylphenyl |
| O | O | isobutyl | R⁷ and R⁸ cyclopropyl | form a | hydrogen |
| O | O | isobutyl | R⁷ and R⁸ cyclopropyl | form a | methyl |
| O | O | benzyl | R⁷ and R⁸ cyclopropyl | form a | benzyl |
| O | C | benzyl | hydrogen | hydrogen | hydrogen |
| O | C | benzyl | hydrogen | hydrogen | methyl |
| O | C | benzyl | hydrogen | hydrogen | benzyl |
| O | C | benzyl | hydrogen | hydrogen | phenyl |
| O | C | benzyl | hydrogen | hydrogen | p-chlorophenyl |
| O | C | benzyl | hydrogen | hydrogen | ethyl |
| O | C | benzyl | hydrogen | hydrogen | m-methylbenzyl |
| O | C | benzyl | hydrogen | hydrogen | p-methylphenyl |
| O | C | benzyl | R⁷ and R⁸ cyclopropyl | form a | hydrogen |
| O | C | benzyl | R⁷ and R⁸ cyclopropyl | form a | methyl |
| O | C | isobutyl | hydrogen | hydrogen | hydrogen |
| O | C | isobutyl | hydrogen | hydrogen | methyl |
| O | C | isobutyl | hydrogen | hydrogen | benzyl |
| O | C | isobutyl | hydrogen | hydrogen | phenyl |
| O | C | isobutyl | hydrogen | hydrogen | p-chlorophenyl |
| O | C | isobutyl | hydrogen | hydrogen | ethyl |
| O | C | isobutyl | hydrogen | hydrogen | m-methylbenzyl |
| O | C | isobutyl | hydrogen | hydrogen | p-methylphenyl |
| O | C | isobutyl | R⁷ and R⁸ cyclopropyl | form a | hydrogen |
| O | C | isobutyl | R⁷ and R⁸ cyclopropyl | form a | methyl |
| NH | C | isobutyl | hydrogen | hydrogen | hydrogen |
| NH | C | isobutyl | hydrogen | hydrogen | methyl |
| NCH₃ | O | isobutyl | hydrogen | hydrogen | benzyl |
| NH | O | isobutyl | hydrogen | hydrogen | phenyl |
| NH | C | isobutyl | hydrogen | hydrogen | p-chlorophenyl |
| NH | C | isobutyl | hydrogen | hydrogen | ethyl |
| NH | O | isobutyl | hydrogen | hydrogen | m-methylbenzyl |
| NH | C | isobutyl | hydrogen | hydrogen | p-methylphenyl |
| NH | O | isobutyl | R⁷ and R⁸ cyclopropyl | form a | hydrogen |
| NH | C | isobutyl | R⁷ and R⁸ cyclopropyl | form a | methyl |
| NH | C | benzyl | hydrogen | hydrogen | hydrogen |
| O | SO | benzyl | hydrogen | hydrogen | methyl |
| O | S | benzyl | hydrogen | hydrogen | benzyl |
| O | SO₂ | benzyl | hydrogen | hydrogen | phenyl |
| O | C | benzyl | hydrogen | hydrogen | p-chlorophenyl |
| O | NH | benzyl | hydrogen | hydrogen | ethyl |
| O | C | benzyl | hydrogen | hydrogen | m-methylbenzyl |
| O | C | benzyl | hydrogen | hydrogen | p-methylphenyl |
| O | C | benzyl | R⁷ and R⁸ cyclopropyl | form a | hydrogen |
| O | C | benzyl | R⁷ and R⁸ cyclopropyl | form a | methyl |
| O | O | isobutyl | methyl | hydrogen | hydrogen |
| O | O | isobutyl | methyl | hydrogen | methyl |
| O | O | isobutyl | methyl | hydrogen | benzyl |
| O | O | isobutyl | methyl | hydrogen | phenyl |
| O | O | isobutyl | methyl | hydrogen | p-chlorophenyl |
| O | O | isobutyl | methyl | hydrogen | ethyl |
| O | O | isobutyl | methyl | hydrogen | m-methylbenzyl |
| O | O | isobutyl | hydrogen | methyl | p-methylphenyl |
| O | O | isobutyl | hydrogen | methyl | hydrogen |
| O | O | isobutyl | hydrogen | methyl | methyl |
| O | O | benzyl | methyl | hydrogen | benzyl |
| O | C | benzyl | methyl | methyl | hydrogen |
| O | C | benzyl | methyl | hydrogen | methyl |
| O | C | benzyl | hydrogen | methyl | benzyl |
| O | C | benzyl | methyl | hydrogen | phenyl |
| O | C | benzyl | methyl | hydrogen | p-chlorophenyl |
| O | C | benzyl | methyl | methyl | ethyl |
| O | C | benzyl | methyl | hydrogen | m-methylbenzyl |
| O | C | benzyl | hydrogen | methyl | p-methylphenyl |
| O | C | benzyl | methyl | methyl | hydrogen |
| O | C | benzyl | methyl | hydrogen | hydrogen |
| O | C | isobutyl | methyl | hydrogen | methyl |
| O | C | isobutyl | methyl | hydrogen | benzyl |
| O | C | isobutyl | hydrogen | methyl | phenyl |
| O | C | isobutyl | hydrogen | methyl | p-chlorophenyl |
| O | C | isobutyl | methyl | hydrogen | ethyl |
| O | C | isobutyl | hydrogen | methyl | m-methylbenzyl |
| O | C | isobutyl | methyl | hydrogen | p-methylphenyl |
| O | C | isobutyl | methyl | methyl | hydrogen |
| O | C | isobutyl | methyl | methyl | methyl |
| NH | C | isobutyl | methyl | hydrogen | hydrogen |
| NH | C | isobutyl | hydrogen | methyl | methyl |
| NCH₃ | O | isobutyl | hydrogen | methyl | benzyl |
| NH | O | isobutyl | methyl | hydrogen | phenyl |
| NH | C | isobutyl | methyl | hydrogen | p-chlorophenyl |
| NH | C | isobutyl | methyl | hydrogen | ethyl |
| NH | O | isobutyl | methyl | hydrogen | m-methylbenzyl |
| NH | C | isobutyl | hydrogen | methyl | p-methylphenyl |
| NH | O | isobutyl | methyl | methyl | hydrogen |
| NH | C | isobutyl | methyl | methyl | methyl |
| NH | C | benzyl | methyl | hydrogen | hydrogen |
| O | SO | benzyl | hydrogen | methyl | methyl |
| O | S | benzyl | hydrogen | methyl | benzyl |
| O | SO₂ | benzyl | methyl | hydrogen | phenyl |
| O | C | benzyl | hydrogen | methyl | p-chlorophenyl |
| O | NH | benzyl | hydrogen | methyl | ethyl |
| O | C | benzyl | hydrogen | methyl | m-methylbenzyl |
| O | C | benzyl | hydrogen | methyl | p-methylphenyl |
| O | C | benzyl | methyl | methyl | hydrogen |
| O | C | benzyl | methyl | methyl | methyl |

The present invention provides compounds for use in a method of alleviating a pathological condition caused by hyperproliferating mammalian cells comprising administering to a subject an effective amount of a pharmaceutical or veterinary composition disclosed herein to inhibit proliferation of the cells. In a preferred embodiment of this invention, the method further comprises administering to the subject at least one additional therapy directed to alleviating the pathological condition. In a preferred embodiment of the present invention, the pathological condition is characterized by the formation of neoplasms. In a further preferred embodiment of the present invention, the neoplasms are selected from the group consisting of mammary, small-cell lung, non-small-cell lung, colorectal, leukemia, melanoma, pancreatic adenocarcinoma, central nervous system (CNS), ovarian, prostate, sarcoma of soft tissue or bone, head and neck, gastric which includes pancreatic and esophageal, stomach, myeloma, bladder, renal, neuroendocrine which includes thyroid and non-Hodgkin's disease and Hodgkin's disease neoplasms.

As used herein "neoplastic" refers to a neoplasm, which is an abnormal growth, such growth occurring because of a proliferation of cells not subject to the usual limitations of growth. As used herein, "anti-neoplastic agent" is any compound, composition, admixture, co-mixture, or blend which inhibits, eleminates, retards, or reverses the neoplastic phenotype of a cell.

Anti-mitotic agents or poisins may be classified into three groups on the basis of their molecular mechanism of action. The first group consists of agents, including colchicine and colcemid, which inhibit the formation of microtubules by sequestering tubulin. The second group consists of agents, including vinblastine and vincristine, which induce the formation of paracrystalline aggregates of tubulin. Vinblastine and vincristine are well known anticancer drugs: their action of disrupting mitotic spindle microtubules preferentially inhibits hyperproliferative cells. The third group consists of agents, including taxol, which promote the polymerization of tubulin and thus stabilizes microtubules.

The exhibition of drug resistance and multiple-drug resistance phenotype by many tumor cells and the clinically proven mode of action of anti-microtubule agents against neoplastic cells necessitates the development of anti-microtubule agents cytotoxic to non-drug resistant neoplastic cells as well as cytotoxic to neoplastic cells with a drug resistant phenotype.

Chemotherapy, surgery, radiation therpy, therapy with biological response modifiers, and immunotherapy are currently used in the treatment of cancer. Each mode of therapy has specific indications which are known to those of ordinary skill in the art, and one or all may be employed in an attempt to achieve total destruction of neoplastic cells. Moreover, combination chemotherapy, chemotherapy utilizing compounds of Formula I or Formula III in combination with other neoplastic agents, is also provided by the subject invention as combination therapy is generally more effective than the use of a single anti-neoplastic agent. Thus, a further aspect of the present invention provides compositions containing a therapeutically effective amount of at least one compound of Formula I or Formula III, including the non-toxic addition salts thereof, which serve to provide the above recited benefits. Such compositions can also be provided together with physiologically tolerable liquid, gel, or solid carriers, diluents, adjuvants and excipients. Such carriers, adjuvants, and excipients may be found in the U.S. *Pharmacopeia,* Vol. **XXII** and National Formulary vol **XVII**, U.S. *Pharmacopeia* Convention, Inc. Rockville, MD (1989). Additional modes of treatment are provided in AHFS Drug Information, 1993 e. by the American Hospital Formulary Service, pp. 522-660. Each of these references are well known and readily available to the skilled artisan.

The present invention further provides that the pharmaceutical composition used to treat neoplastic disease contains at least one compound of Formula I or Formula III and at least one additional anti-neoplastic agent. Anti-neoplastic agents which may be utilized in combination with Formula I or Formula III compounds include those provided in the Merck Index **11**, pp 16-17, Merck & Co., Inc. (1989). The Merck Index is widely recognized and readily available to the skilled artisan.

In a further embodiment of this invention, antineoplastic agents may be antimetabolites which may include those selected from the group consisting of methotrexate, 5-fluorouracil, 6-mercaptopurine, cytosine, arabinoside, hydroxyurea, and 2-chlorodeoxyadenosine. In another embodiment of the present invention, the anti-neoplastic agents contemplated are alkylating agents which may include those selected from the group consisting of cyclophosphamide, mephalan, busulfan, paraplatin, chlorambucil, and nitrogen mustard. In a further embodiment, the anti-neoplastic agents are plant alkaloids which may include those selected from the group consisting of vincristine, vinblastine, taxol, and etoposide. In a further embodiment, the anti-neoplastic agents contemplated are antibiotics which may include those selected from the group consisting of doxorubicin, daunorubicin, mitomycin C, and bleomycin. In a further embodiment, the anti-neoplastic agents contemplated are hormones which may include those selected from the group consisting of calusterone, diomostavolone, propionate, epitiostanol, mepitiostane, testolactone, tamoxifen, polyestradiol phosphate, megesterol acetate, flutamide, nilutamide, and trilotane.

In a further embodiment, the anti-neoplastic agents contemplated include enzymes which may include those selected from the group consisting of L-Asparginase and aminoacridine derivatives such as, but not limited to, amsacrine. Additional anti-neoplastic agents include those provided by Skeel, Roland T., "Antineoplastic Drugs and Biologic Response Modifier: Classification, Use and Toxicity of Clinically Useful Agents" Handbook of Cancer Chemotherapy (3rd ed.), Little Brown & Co. (1991).

These compounds and compositions can be administered to mammals for veterinary use. For example, domestic animals can be treated in much the same way as a human clinical patient. In general, the dosage required for therapeutic effect will vary according to the type of use, mode of administration, as well as the particularized requirements of the individual hosts. Typically, dosages will range from about 0.001 to 1000 mg/kg, and more usually 0.01 to 10 mg/kg of the host body weight. Alternatively, dosages within these ranges can be administered by constant infusion over an extended period of time, usually exceeding 24 hours, until the desired therapeutic benefits are obtained. Indeed, drug dosage, as well as route of administration, must be selected on the basis of relative effectiveness, relative toxicity, growth characteristics of tumor and effect of Formula I or Formula III compound on cell cycle, drug pharmacokinetics, age, sex, physical condition of the patient and prior treatment.

The compound of Formula I or Formula III, with or without additional anti-neoplastic agents, may be formulated into therapeutic compositions as natural or salt forms. Pharmaceutically acceptable non-toxic salts include base addition salts which may be derived from inorganic bases such as for example, sodium, potassium, ammonium, calcium, or ferric hydroxides, and such organic bases as isopropylamine, trimethylamine, 2-ethylamino ethanol, histidine, procaine, and the like. Such salts may also be formed as acid addition salts with any free cationic groups and will generally be formed with inorganic acids such as for example, hydrochloric or phosphoric acids or organic acids such as acetic, oxalic, tartaric, mandelic, and the like. Additional excipients which further the invention are provided to the skilled artisan for example in the U.S. *Pharmacopeia*.

The suitability of particular carriers for inclusion in a given therapeutic composition depends on the preferred route of administration. For example, anti-neoplastic compositions may be formulated for oral administration. Such compositions are typically prepared as liquid solution or suspensions or in solid forms. Oral formulation usually include such additives as binders, fillers, carriers, preservatives, stabilizing agents, emulsifiers, buffers, mannitol, lactose, starch, magnesium stearate, sodium saccharin, cellulose, magnesium carbonate, and the like. These compositions may take the form of solutions, suspensions, tablets, pills, capsules, sustained relsease formulations, or powders, and typically contain 1% to 95% of active ingedient. More preferably, the composition contains from about 2% to about 70% active ingredient.

Compositions of the present invention may be prepared as injectables, either as liquid solutions, suspensions, or emulsions; solid forms suitable for solution in or suspension in liquid prior to injection. Such injectables may be administered subcutaneously, intravenously, intraperitoneally, intramuscularly, intrathecally, or intrapleurally. The active ingredient or ingredients are often mixed with diluents, carriers, or excipients which are physiologically tolerable and compatible with the active ingredient(s). Suitable diluents and excipients are for example, water, saline, dextrose, glycerol, or the like and combinations thereof. In addition, if desired, the compositions may contain minor amounts of auxilary substances such as wetting or emulsifying agents, stabilizing or pH buffering agents.

The invention further provides Formula I or Formula III compounds for use to inhibit the proliferation of mammalian cells by contacting these cells with a Formula I or Formula III compound in an amount sufficient to inhibit the proliferation of the mammalian cell. A preferred embodiment is a method to inhibit the proliferation of hyperproliferative mammalian cells. For purposes of this invention "hyperproliferative mammalian cells" are mammalian cells which are not subject to the characteristic limitations of growth (programmed cell death for example). A further preferred embodiment is when the mammalian cell is human. The invention further provides contacting the mammalian cell with at least one Formula I or Formula III compound and at least one anti-neoplastic agent. The types of anti-neoplastic agents contemplated are discussed *supra.*

The invention further provides Formula I or Formula III compounds for use to inhibit the proliferation of hyperproliferative cells with drug-resistant phenotypes, including those with multiple drug-resistant phenotypes, by contacting said cell with a compound of Formula I or Formula III in an amount sufficient to inhibit the proliferation of a hyperproliferative mammalian cell. A preferred embodiment is when the mammalian cell is human. The invention further provides contacting a Formula I or Formula III compound and at least one additional anti-neoplastic agent, discussed *supra.*

The invention provides a method for alleviating pathological conditions caused by hyperproliferating mammalian cells for example, neoplasia, by administering to a subject an effective amount of a pharmaceutical composition containing Formula I or Formula III compound to inhibit the proliferation of the hyperproliferating cells. As used herein "pathological condition" refers to any pathology arising from the proliferation of mammalian cells that are not subject to the normal limitations of growth. Such proliferation of cells may be due to neoplasms as discussed *supra.*

In a further preferred embodiment the neoplastic cells are human. The present invention discloses methods of alleviating such pathological conditions utilizing a compound of Formula I or Formula III in combination with other therapies, as well as other anti-neoplastic agents.

The effectiveness of the claimed compounds can be assessed using standard methods known to the skilled artisan. One such study provided the following results:

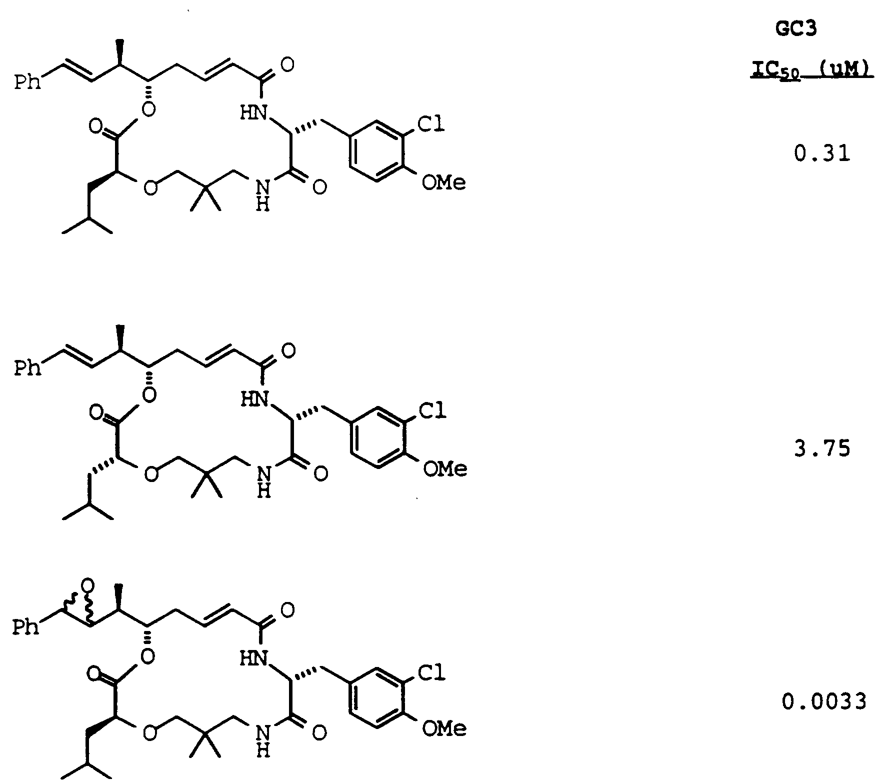

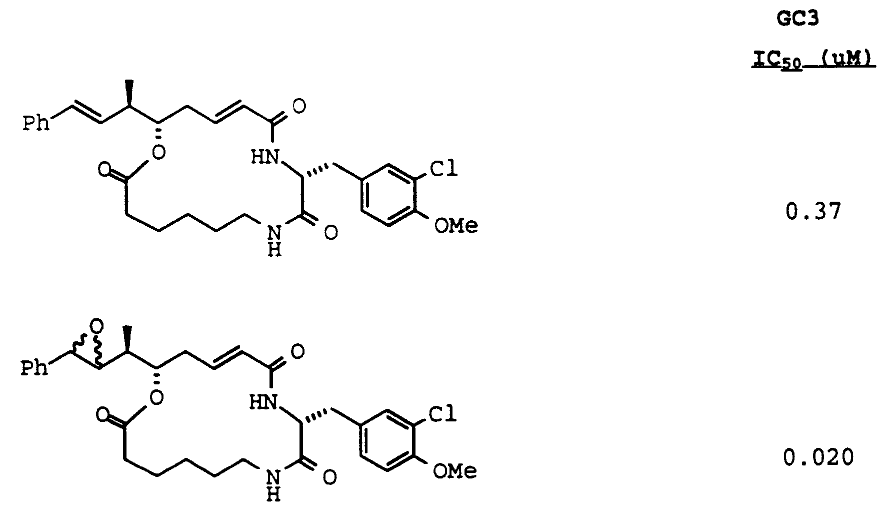

The compounds are screened for minimum inhibitory concentrations against KB, a human nasopharyngeal carcinoma cell line, LoVo, a human colorectal adenocarcinoma cell line.

The Corbett assay, see Corbett, T.H. et al. Cytotoxic Anticancer Drugs: Models and Concepts for Drug Discovery and Development, pp 35-87, Kluwer Academic Publishers: Norwell, 1992. see also, Valeriote, et al. Discovery and Development of Anticancer Agents; Kluwer Academic Publishers, Norwell, 1993.

The most active compounds are further evaluated for cytotoxicity against four different cell types, for example a murine leukemia, a murine solid tumor, a human solid tumor, and a low malignancy fibroblast using the Corbett assay.

The compounds are further evaluated against a broad spectrum of murine and human tumors implanted in mice, including drug resistant tumors.

Tumor burden (T/C) (mean tumor burden in treated animals verses mena tumor burden in untreated animals) are used as a further assessment. T/C values that are less than 42% are considered to be active by National Cancer Institute Standards; T/C values less than 10% are considered to have excellent activity and potential clinical activity by National Cancer Institute standards.

Evaluation of compounds of Formula I and Formula III suggest that the compounds can be useful in the treatment methods described herein. Further, the compounds will be useful for disrupting the microtubule system.

Compounds of this invention can be prepared as illustrated using the following schemes.

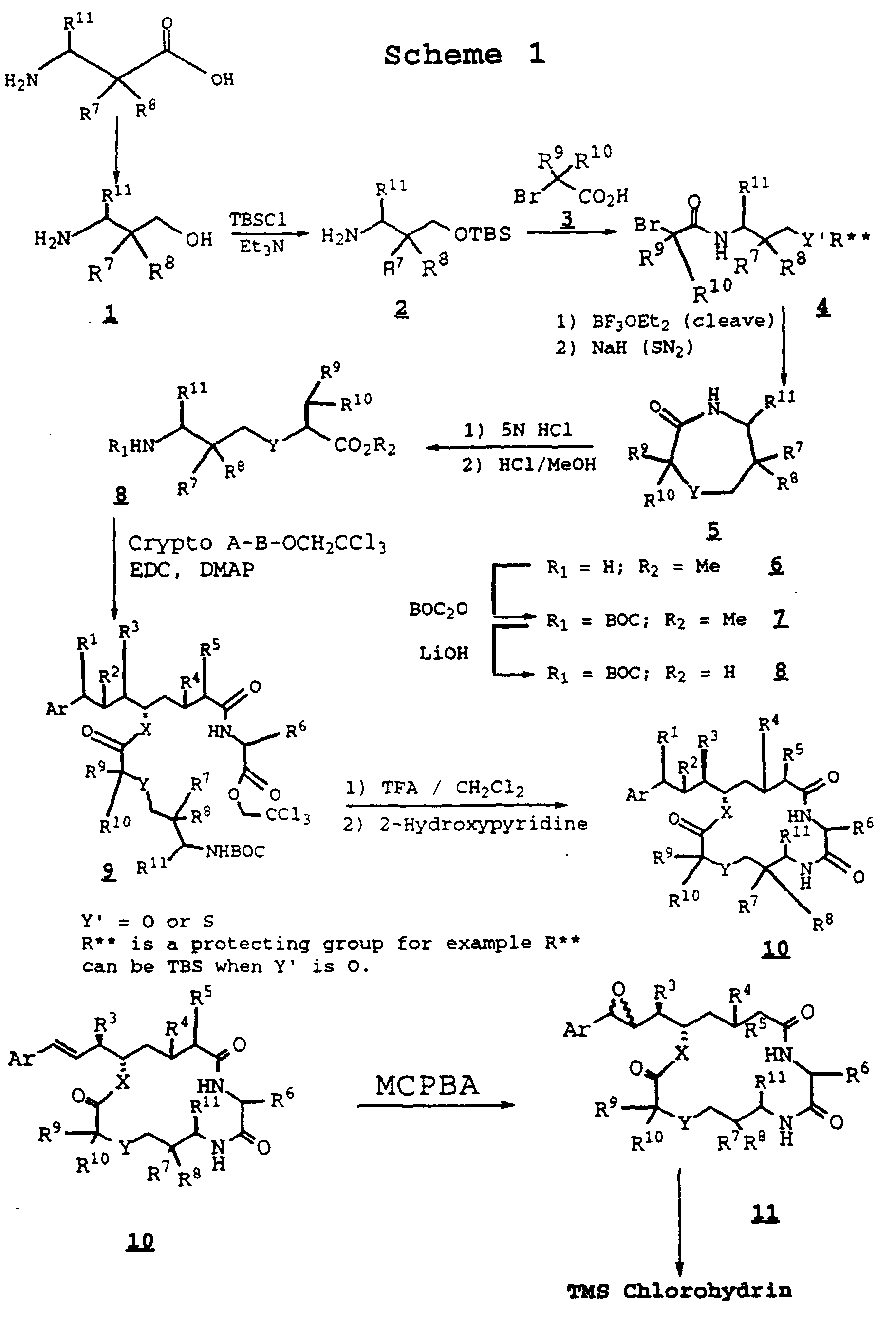

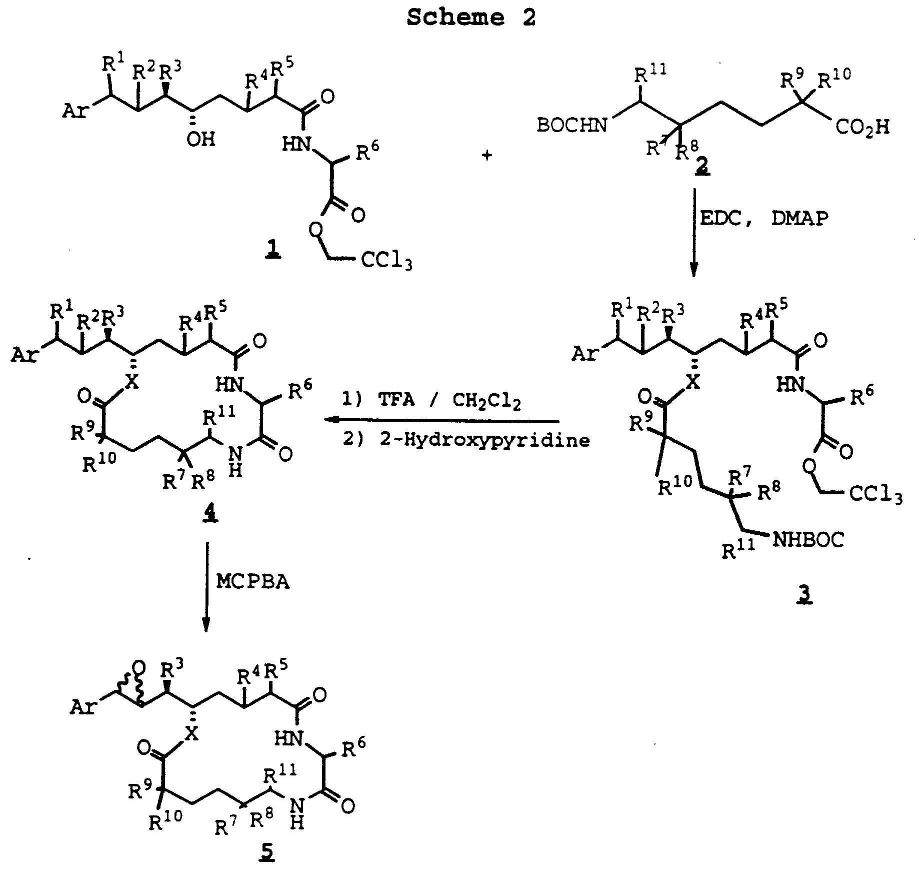

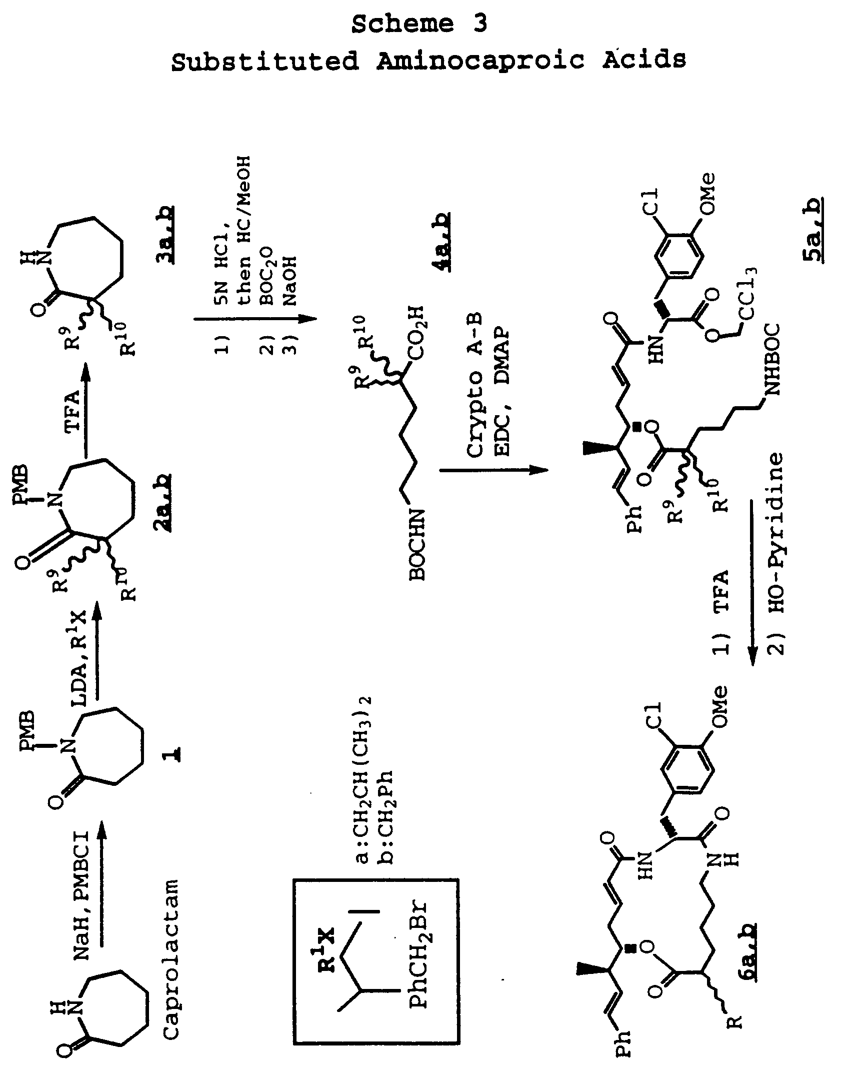

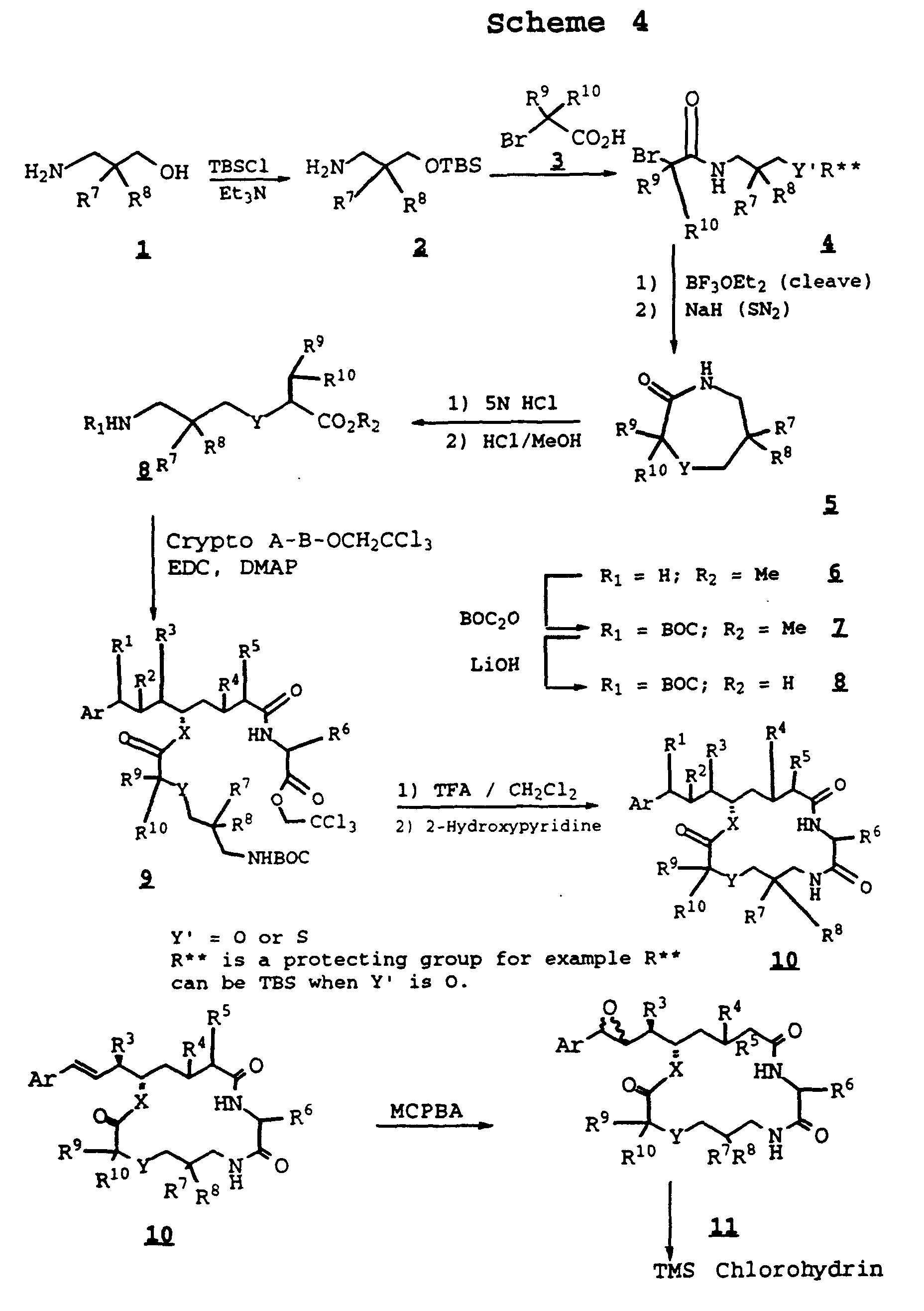

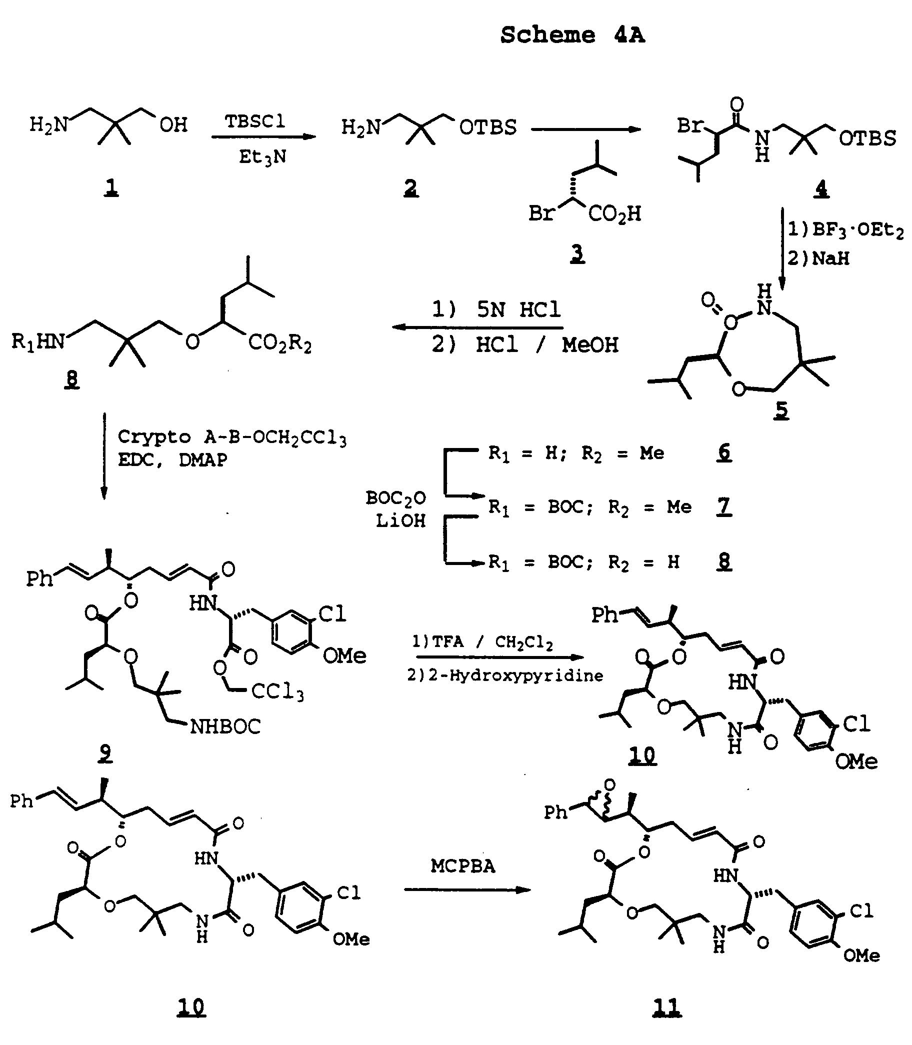

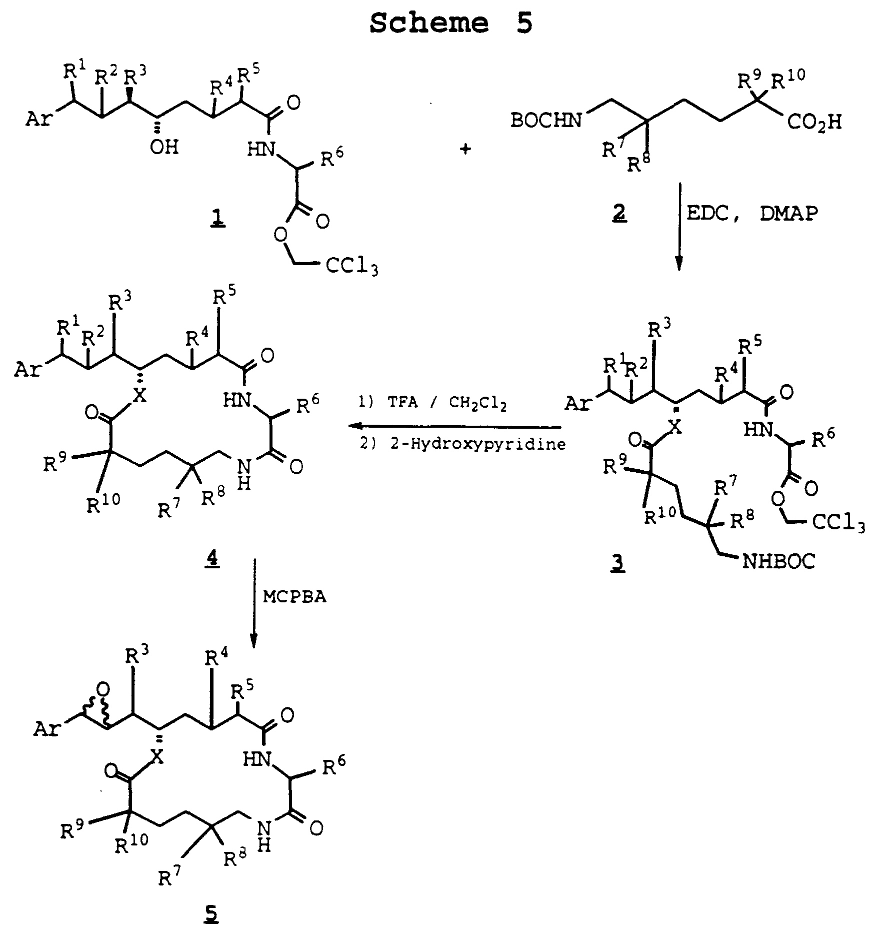

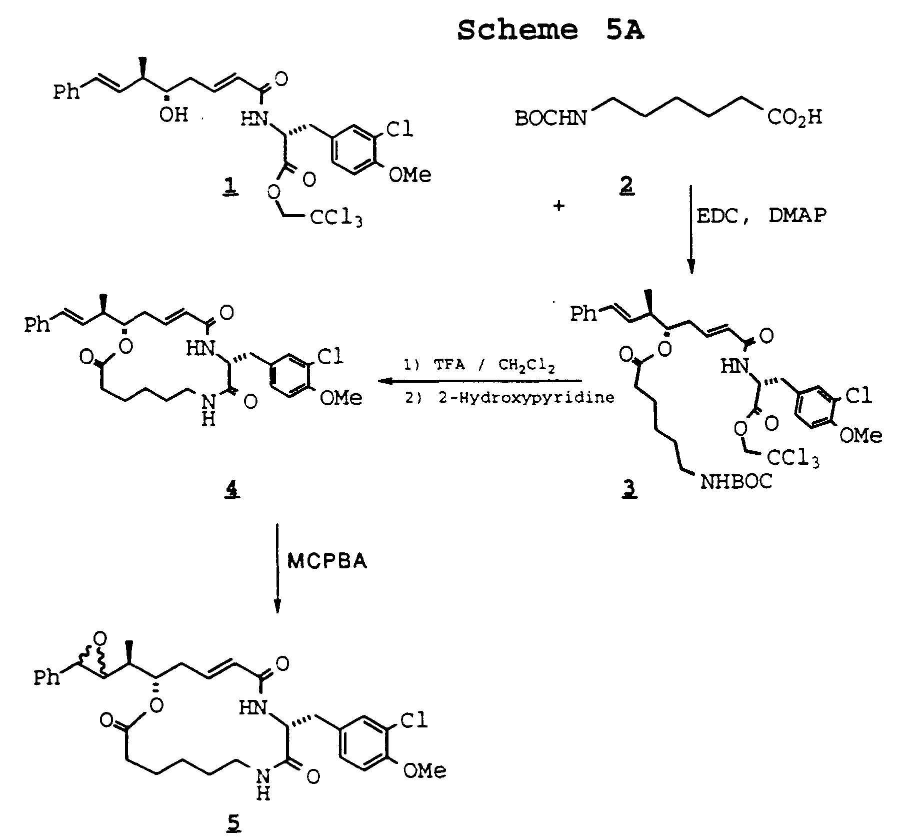

The crypto-A-B trichloroethyl ester can be prepared using information known in the literature; however, the following method is provided for the convenience of the artisan:

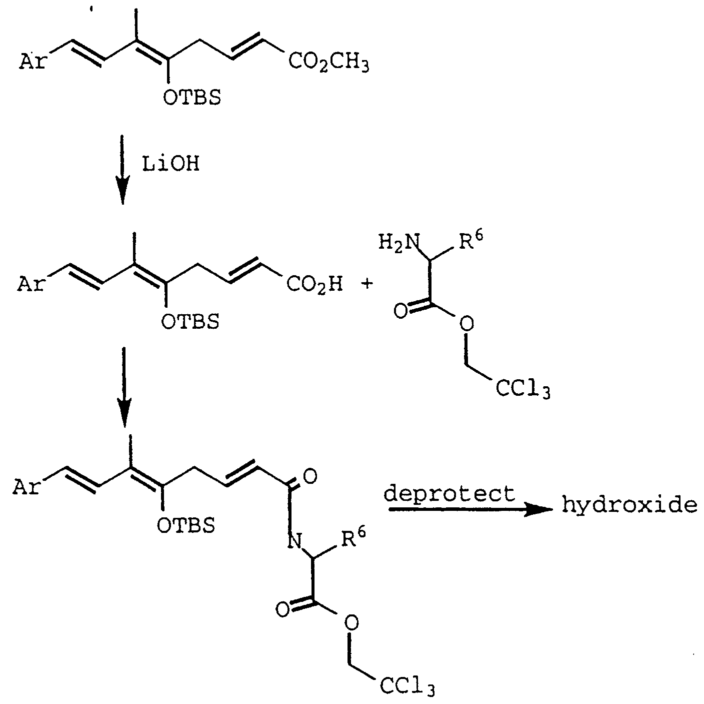

The artisan can utilize appropriate starting materials and reagents to prepare desired compounds using the guidance of the previous schemes and following examples.

The artisan can utilize appropriate starting materials and reagents to prepare desired compounds using the guidance of the previous schemes and following examples.

To further illustrate the invention the following examples are provided.

### Preparation 1

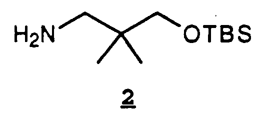

A solution containing 10.0 g (96.9 mmol) of 2,2-dimethyl-3-amino-1-propanol and 16.2 mL (116 mmol) of triethylamine in 250 mL of CH₂Cl₂ was stirred at 0°C. To this solution was added 474 mg (4 mol %) of dimethylaminopyridine (hereinafter referred to as "DMAP") and 16.1 g (107 mmol) of *tert*-butyldimethylsilyl chloride (TBSCl). The reaction was allowed to warm to 25°C over one hour and stirred at that temperature for an additional 18 h. The reaction was concentrated *in vacuo* and dissolved in 150 mL of ethyl acetate. The organic solution was wahed three times with aqueous sodium bicarbonate solution, dried over sodium sulfate and concentrated in vacuo to give 15.0 g a light yellow oil, which was characterized as **2**. Mass Spec. (FD⁺) m/e 218 (M+1⁺).

### Preparation 2

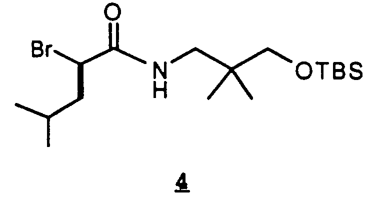

A solution of 3.25 g (16.7 mmol) of (*R*)-2-bromo-4-methylpentanoic acid¹ in 200 mL of CH₂Cl₂ was stirred at 0°C as 2.25 g (16.7 mmol) of hydroxybenzotriazole (HOBt) was added, followed by the addition of 3.19 g (16.7 mmol) of 1-ethyl-3-(3-dimethylaminopropyl)carbodiimide (EDC). The reaction was stirred for 1.5 h at 0°C, after which 3.62 g (16.7 mmol) of the protected amino alcohol **2** in 25 mL of CH₂Cl₂ was added dropwise over about 5 min. The reaction was stirred an additional 2 h at 0°C, and poured into 200 mL of a saturated aqueous sodium bicarbonate solution. The organic phase was washed twice with saturated aqueous sodium bicarbonate, twice with 10% aqueous citric acid and twice with brine. The organic solution was dried over sodium sulfate and concentrated *in vacuo* to give 4.90 g (74 %) of a clear oil, which was characterized as **4**. Mass Spec. (FD⁺) m/e 394, 396 (M+1⁺).

### Preparation 3

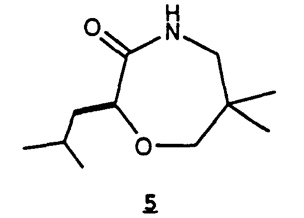

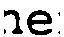

To a solution containing 1.00 g (2.54 mmol) of the protected amide **4** in 20 mL of CH₂Cl₂ at 0°C was added 0.34 mL (2.79 mmol) of boron trifluoride etherate (BF₃-OEt₂) and the reaction was warmed to 25°C. After stirring for one hour at 25°C, an additional 0.34 mL of boron trifluoride etherate was added and the reaction was stirred an additional one hour at 25°C. The reaction was poured into 100 ml water, and diluted with an additional 50 mL CH₂Cl₂. The organic solution was washed twice with saturated sodium bicarbonate solution, dried over sodium sulfate and concentrated *in vacuo* to give 560 mg (79 %) of the intermediate bromo alcohol. A solution containing 500 mg (1.78 mmol) of this material in 10 mL of anhydrous tetrahydrofuran (THF) was added to a suspension of 86 mg (2.14 mmol) of sodium hydride (60 % in mineral oil) at -78°C. The reaction was allowed to slowly warm to 25°C over one hour and quenched with 1N HCl. The organic phase was separated, dried over sodium sulfate and concentrated *in vacuo.* This crude material was purified by flash chromatography on silica gel, using ethyl acetate as the eluent to give 185 mg (52 %) of a clear oil, which was characterized as **5**. Mass Spec. (FD⁺) m/e 199 (M⁺).

### Preparation 4

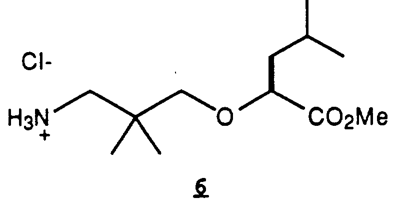

A suspension of 1.69 g (8.48 mmol) of lactam **5** in 20 mL 5N HCl was heated to reflux for 2 h, cooled to 25°C and concentrated *in vacuo* to dryness. The residue was dissolved in 200 mL of methanolic HCl and stirred for 24 h at 25°C and concentrated *in vacuo* to give 2.20 g (97 %) of a white amorphous solid, which was characterized as **6**. Mass Spec. (FD⁺) m/e 232 (M+1⁺)

### Preparation 5

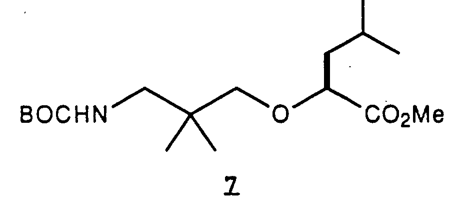

A solution of 1.66 g (6.20 mmol) of the HCl salt **6** was dissolved in 100 mL of CH₂Cl₂ and stirred at 25°C as 1.03 mL (7.44 mm) of triethylamine was added dropwise, followed by the addition of 1.62 g (7.44 mmol) of di-*tert*-butyl dicarbonate (BOC₂O). The reaction was stirred at 25°C for 2 hours, diluted with 200 mL ethyl acetate and washed twice with 1N HCl. The organic phase was dried over sodium sulfate and concentrated *in vacuo* to give crude **7**, which was purified by flash chromatography on silica gel, using 50% hexane-ethyl acetate as the eluent. The major fraction was concentrated *in vacuo* to give 1.80 g (88%) of a clear oil, which was characterized as **7**. Mass Spec. (FD⁺) m/e 331 (M⁺).

### Preparation 6

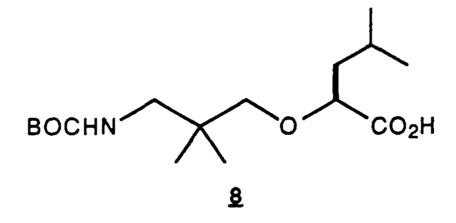

To a solution of 1.00 g (3.02 mmol) of **7** in 10 mL of 50 % THF-water was added 253 mg (6.04 mmol) of lithium hydroxide hydrate at 25°C. The reaction was stirred for 1 h at 25°C, poured into 1N HCl and extracted with ethyl acetate. The organic extracts were dried and concentrated in vacuo to give 905 mg (94%) of a white amorphous solid, which was characterized as **8**. Mass Spec. (FD⁺) m/e 318 (M+1⁺).

### Preparation 7

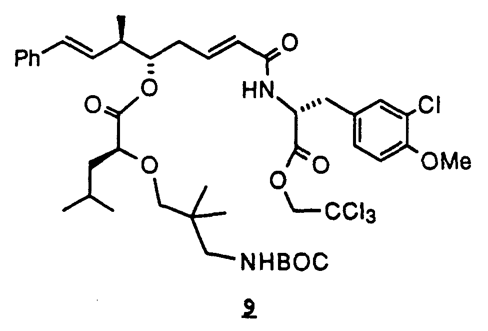

To a solution containing 323 mg (1.02 mmol) of **8** in 20 mL of CH₂Cl₂ was added 195 mg (1.02 mmol) of 1-ethyl-3-(3-dimethylaminopropyl)carbodiimide (EDC), followed by 10 mg of dimethylaminopyridine (hereinafter "DMAP"). The reaction was stirred at 25°C for 30 min, after which 500 mg (0.848 mmol) of cryptophycin A-B trichloroethyl ester was added. The reaction was stirred an additional 15 min, washed with 1N HCl, dried over sodium sulfate and concentrated *in vacuo.* The crude material was purified by flash chromatography using 25% ethyl acetate-hexane as the eluent. The major fraction was concentrated *in vacuo* to give 451 mg (67%) of a white amorphous solid, which was characterized as **9**. Mass Spec. (FD⁺) m/e 886 (M⁺)

### Preparation 8

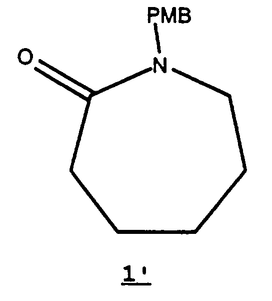

A 3.36g (60%in oil , 0.084 moles) sample of sodium hydride was added to a solution of 7.91g (0.07 moles) of e-Caprolactam in 220 mL of dimethylformamide and stirred for 30 minutes. 13.34 mL (0.098 moles) of p-methoxybenzoyl chloride was added and the reaction was stirred for 18 hours. The reaction was poured into 200 mL of 1N HCl and extracted two times with ethyl acetate. The organic layer was washed with water, dried over sodium sulfate and concentrated *in vacuo* to give 18.4g (100 %) of a yellow oil which was characterized as **1'.** (CDCl₃, 300 MHz) d 1.2-1.9 (series of m, 6H); 2.6(m, 2H); 3.2-3.4 (m, H-2); 3.8 (s, 3H, CH₃); 4.5 (s, 2H); 6.8-7.0 (m, 2H); 7.15-7.4 (m, 2H)

### Preparation 9

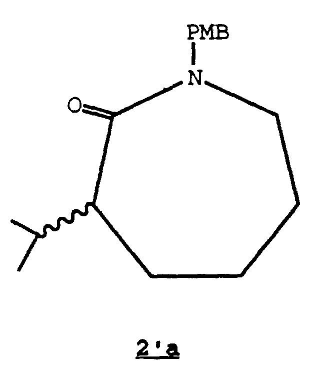

To a solution of 4.33 g (31 mmol) of diisopropylamine in 120 mL of tetrahydrofuran at ice bath temperature was added 19.3 mL (31 mmol) of butyl lithium (1.6M in hexanes). After 15 minutes the reaction was cooled to -70°C and a solution 6.0g (26 mmol) of the protected lactam **1'** in 20 mL of tetrahyrofuran was added. After 25 minutes at -70°C 3.55 mL (31 mmol) of 1-iodo-2-methylpropane.were added and the reaction was allowed to warm to 25°C After 3 hours, 300 mL of 1.0N HCl were added and the aqueous layer was extracted with ethyl acetate. The organic layer was dried over sodium sulfate, and concentrated *in vacuo* to yield 9.0 g ( 100%) of a pale oil which was characterized as **2'a**. (CDCl3, 300 MHz) d 0.9 (d, J= 6.6 Hz 6H); 1.2-1.95(series of multiplets, 9H); 2.55 (m, 1H); 3.1-3.24 (m, 1H); 3.45 (m, 1H); 3.79 (s, 3H, CH3); 4.45 (d, J= 14.5 Hz,1H); 4.6(d, J= 14.4 Hz, 1H); 6.84(d, J= 8.3 Hz, 2H); 7.2(d, J= 8.4Hz, 2H).

### Preparation 10

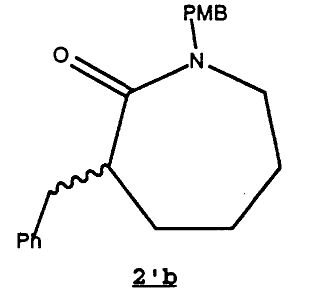

To a solution of 4.33 g (31 mmol) of diisopropylamine in 120 mL of tetrahydrofuran at ice bath temperature was added 19.3 mL (31 mmol) of butyl lithium (1.6M in hexanes). After 15 minutes the reaction was cooled to -70°C and a solution of 6.0 g (26 mmol) of the protected lactam **1'** in 20 mL of tetrahyrofuran was added. After 25 minutes at -70°C 3.67 mL ( 31 mmol) of benzyl bromide.were added and the reaction was allowed to warm to 25°C After 3 hours, 300 mL of 1.0N HCl were added and the aqueous layer was extracted with ethyl acetate. The organic layer was dried over sodium sulfate, and concentrated *in vacuo* to yield 8.9 g ( 100%) of a pale oil which was characterized as **2'b**. Mass Spec. (FD⁺) m/e 323 (M⁺)

### Preparation 11

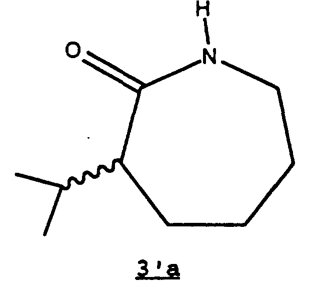

### Preparation 12

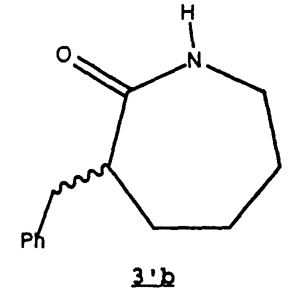

**3'b** was prepared substantially according to the method for the preparation of **3'a**. Mass Spec. (FD⁺ m/e 203 (M⁺).

### Preparation 13

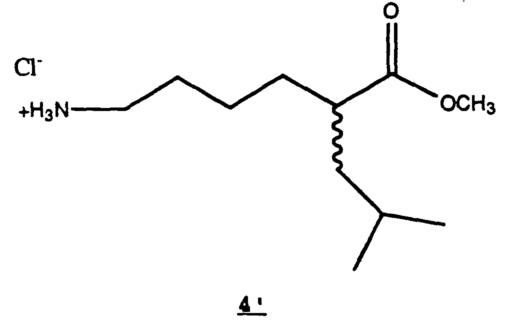

A suspension of 0.3 g (1.8 mmol) of deblocked lactam **3'a** (Preparation 11) in 8.0 mL of 5N hydrochloric acid was refluxed for 4 hours. The reaction was concentrated to a residue *in vacuo.* The residue was dissolved in 25 mL of methanolic HCl and stirred 18 hours at 25°C and concentrated *in vacuo* to give 0.28 g (42%) of an amorphous white solid which was characterized as **4'a**. (CDO₃D, 300 MHz) d 0.95 (m, 6H); 1.2-1.8 (series of multiplets, 10H) ; 2.94 (m, 2H); 3.68 (m, 3H, CH₃) ; 4.9 (m, 3H).

### Preparation 14

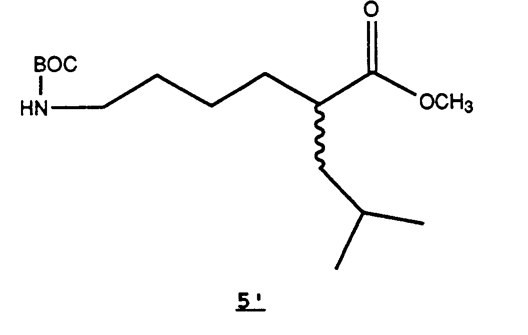

To a solution of 0.57 g (2.4 mmol) of **4'** (Preparation 13) in 10 mL of methylene chloride was added 0.4 mL (2.9 mmol) of triethylamine followed by 0.63 g (2.9 mmol) of di-tert-butyldicarbonate. The reaction was stirred for 18 hours at 25°C diluted with 50 mL of ethyl acetate and washed first with 1N HCl followed by saturated sodium bicarbonate and dried over sodium sulfate. The solution was concentrated *in vacuo* to a residue which was purified by flash chromatography on silica gel using hexane-ethyl acetate 6-1 as eluent to yield 0.44 g (56%) of a clear oil which was characterized as **5'** Mass Spec. (FD⁺) m/e 302 (M⁺).

### Preparation 15

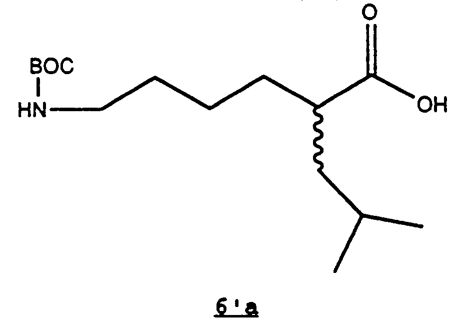

A solution of 0.44 g (1.46 mmol) of **5'** (Preparation 14) was dissolved in 20 mL of methanol and 20 mL of 2N sodium hydroxide and refluxed for two hours. The methanol was removed in vacuo and the aqueous layer was acidified with 1N hydrochloric acid and extracted with ethyl acetate. The organic layer was dried over sodium sulfate and concentrated in vacuo to yield ) 0.55 g (100%)of an oil which was characterized as **6'**. Mass Spec. (FD⁺) m/e 288 (M⁺).

### Preparation 16

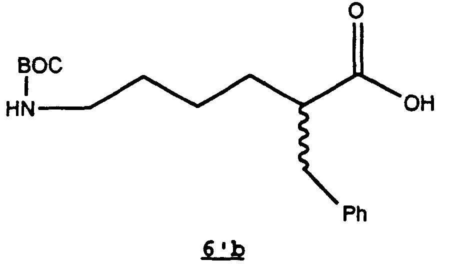

**6'b** was prepared substantially according to the method for the preparation of **6'a.** Mass Spec. (FD⁺ m/e 321 (M⁺).

### Preparation 17

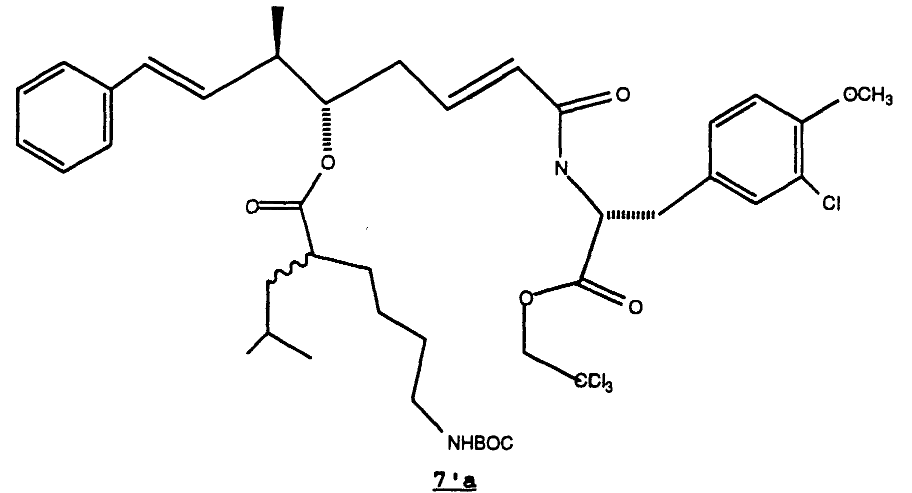

To a solution of 0.27 g (0.94 mmol) of **6'a** (Preparation 15) in 10 mL of methylene chloride was added 0.162 g (0.85 mmol) of 1-ethyl-3-(3-dimethylaminopropyl)carbodiimide (EDC), followed by 10 mg of dimethylaminopyridine. The reaction was stirred at 25°C for 30 minutes after which 0.498 g (0.85 mmol) of cryptophycin A-B trichloroethy ester was added. The reaction was refluxed for 24 hours after which the solvent was removed in vacuo and the residue was dissolved in ethyl acetate and washed with 1N HCl followed by sodium bicarbonate solution and finally , brine. The solvent was removed in vacuo to yield a residue which was purified by flash chromatography on silica gel using hexane-ethyl acetate 2-1 as eluent to yield 0.28 g (35 %) of a white residue which was characterized as **7'a** Mass Spec. (FD⁺) m/e 858 (M⁺)

### Preparation 18

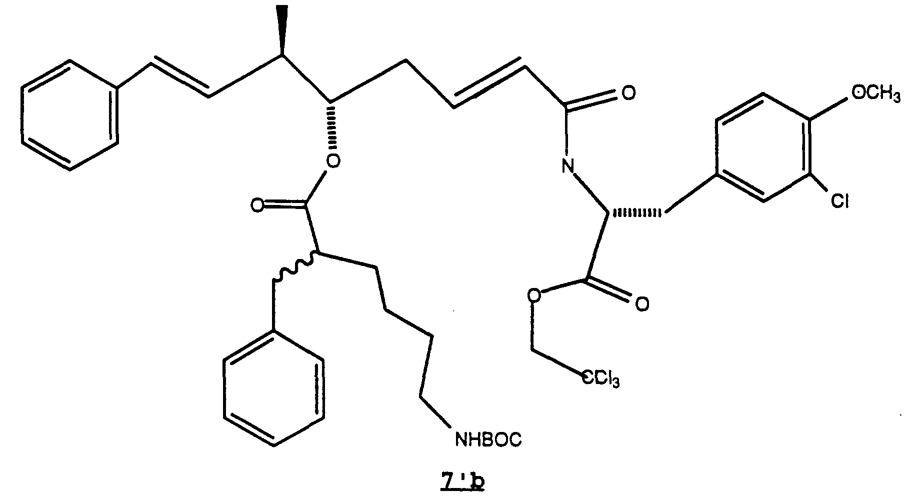

**7'b** was prepared substantially according to the method for the preparation of **7'a**. Mass Spec. (FD⁺ m/e 892 (M⁺).

### Preparation 19

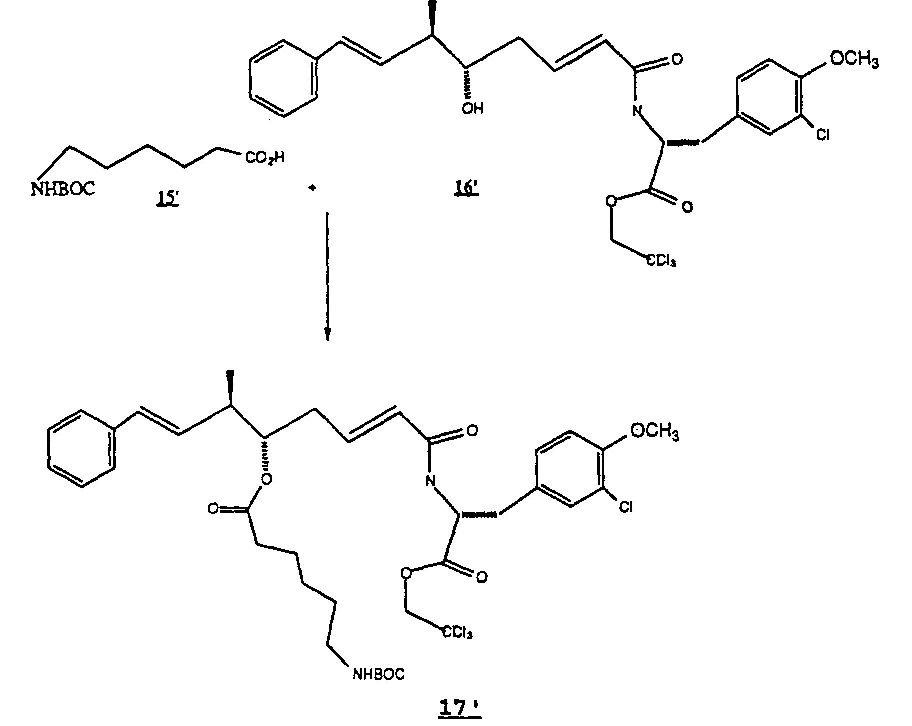

To a solution of 341 mg (1.47 mmol) of 6-BOC-aminocaproic acid (15') in 25 mL of CH₂Cl₂ was added 283 mg (1.47 mmol) of 1-ethyl-3-(3-dimethylaminopropyl)carbodiimide (EDC), followed by 10 mg (catalytic) of 4-dimethylaminopyridine (DMAP) at 25°C. The reaction was stirred at 25°C for 30 min. and 290 mg (0.492 mmol) of cryptophycin A-B trichloroethyl ester (**16'**) was added. The reaction was stirred an additional 15 min, washed with 1N HCl, dried over sodium sulfate and concentrated *in vacuo.* The crude material was purified by flash chromatography using 25% ethyl acetate-hexane as the eluent. The major fraction was concentrated *in vacuo* to give 205 mg (52%) of a white amorphous solid, which was characterized as **17'**. Mass Spec. (FD⁺) m/e 802 (M⁺).

### Example 1

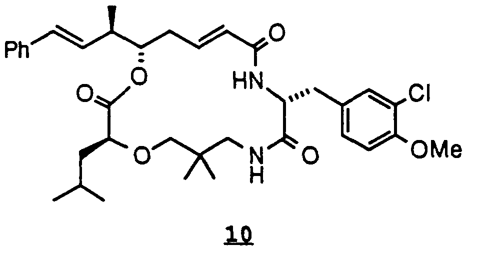

To a solution of 426 mg (0.537 mmol) of **9** (Preparation 7) in 5 mL CH₂Cl₂ was added 2 mL of trifluoroacetic acid (TFA). The reaction was strirred at 25°C for 30 min, after which it was poured into 25 mL of a saturated aqueous sodium bicarbonate solution. An additional 25 mL of CH₂Cl₂ was added and the organic phase was washed with 1N NaOH, dried over sodium sulfate and concentrated *in vacuo.* This crude material was dissolved in 10 mL of toluene and stirred at 25°C as 255 mg (2.68 mmol) of 2-hydroxypyridine was added. The reaction was stirred at 25°C for 18 h. The reaction was diluted with 50 mL of ethyl acetate and the organic phase washed twice with 1N HCl, twice with brine and twice with saturated sodium bicarbonate solution. The organic solution was dried over sodium sulfate and concentrated *in vacuo.* The crude material was purified by flash chromatography on silica gel using 50% ethyl acetate-hexane as the eluent. Two fractions were collected, which correspond to the epimers at the isobutyl substitution site adjacent to the ether moiety. The major fraction was concentrated *in vacuo* to give 106 mg (31%) of a white amorphous solid, which was characterized as **10**. Mass Spec. (FD⁺) m/e 638 (M⁺).

### Example 2

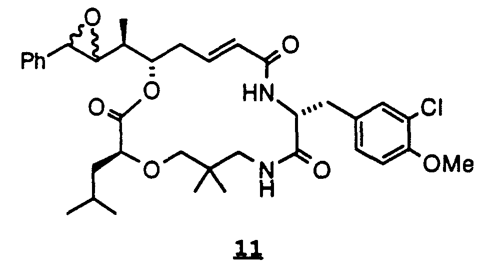

To a solution containing 47 mg (0.074 mmol) of **10** (Example 1) in 10 mL of CH₂Cl₂ was added 22.3 mg (0.11 mmol) of 85% *m*-chloroperbenzoic acid (mCPBA). The reaction was strirred at 25°C and monitored by HPLC (reverse phase, C₁₈, 70% acetonitrile-water). After about 5 h, the reaction slowed and an additional 44 mg *m*CPBA was added. The reaction was stirred at 25°C and after another 12 h, was complete. The reaction was diluted with 25 mL CH₂Cl₂ and washed twice with a saturated sodium meta-bisulfite solution and twice with a saturated sodium bicarbonate solution. The organic layer was dried and concentrated *in vacuo* to give 38 mg (79%) of a white amorphous solid, which was charaterized as a 2:1 mixture of diastereomeric epoxides of **11**. HPLC (Reverse Phase, C18, 70% acetonitrile-water, isocratic, 1.0 ml/min.). Retention Times: Major Isomer: 9.58 min. Minor Isomer: 10.28 min.

### Example 3

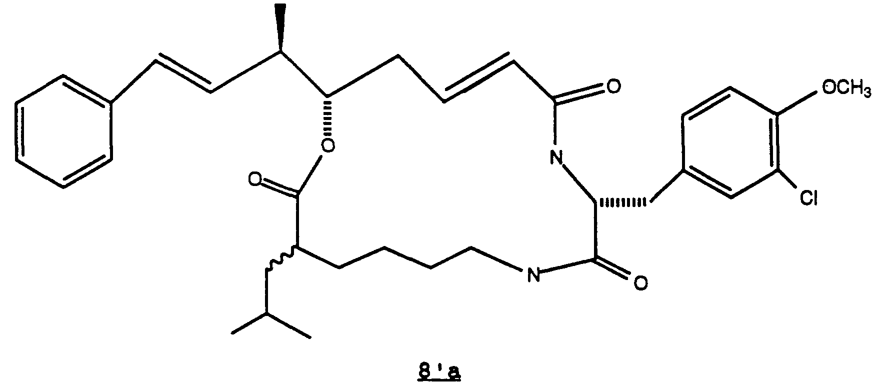

A solution of 0.11 g (0.128 mmol) of **7'** (Preparation 17) and 2.0 mL of trifluoroacetic acid in 2.0 mL of methylene chloride was stirred at 25°C for 45 minutes. The solvent was removed *in vacuo* and the residue was dissolved in 20 mL of methylene chloride, washed with sodium bicarbonate solution and dried over sodium sulfate. After the solvent was removed *in vacuo,* the residue was dissolved in 10 mL of toluene .and 0 061 g (0.64 mmol) of 2-hydroxypyridine was added. After stirring 18 hours at 25°C, the solvent was removed in vacuo, the residue dissolved in ethyl acetate and washed with 1N HCl then sodium bicarbonate solution, then brine, and dried over sodium sulfate. The solvent was removed *in vacuo* and the residue was purified by flash chromatography on silica gel using hexane-ethyl acetate 1-1 as eluent to yield 0.032 g (41%) of a white residue which was characterized as **8'a** Mass Spec. (FD⁺) m/e 609 (M⁺).

### Example 4

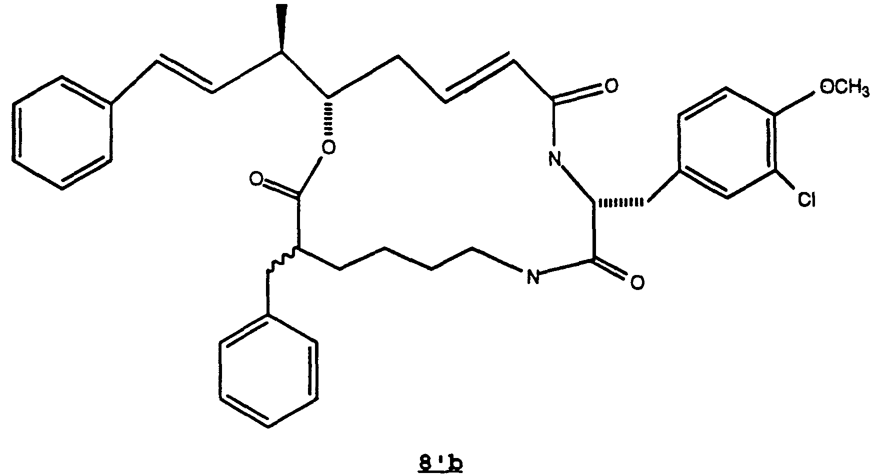

**8'b** was prepared according to the method for the preparation of **8'a**. Mass Spec. (FD⁺) m/e 642 (M⁺).

### Example 5

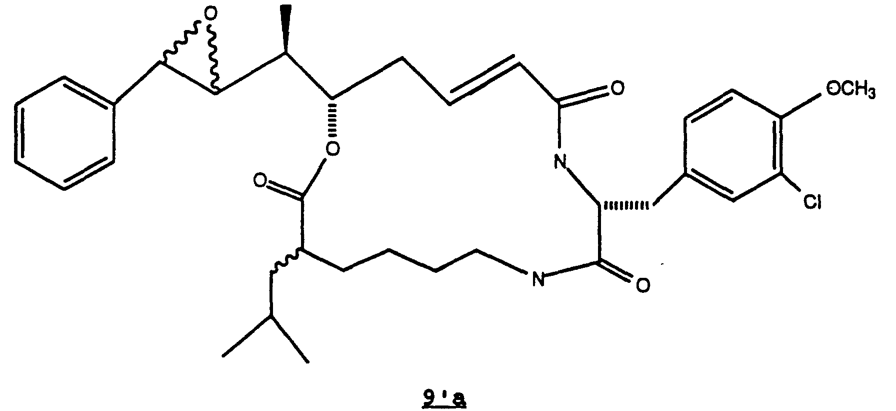

To a solution containing 26 mg (0.043 mmol) of **8'a** (Example 3) in 5.0 mL of methylene chloridewas added 8.1 mg (0.047 mmol) of *m*-chloroperbenzoic acid. The reaction was stirred 18 hours at 25°C and monitored by HPLC (reverse phase, C18, 70% acetonitrile-water). An additional 8.1 mg (0.047 mmol) of *m*-chloroperbenzoic acid was added and the reaction was stirred another 18 hours. The reaction was diluted with 10 mL of methylene chloride and washed with a saturated sodium bisulfite solution followed by sodium bicarbonate solution. The organic layer was dried over sodium sulfate and concentrated in vacuo to give 27 mg (100%) of a white residue which was characterized as a mixture of diastereomeric epoxides of **9'a** Mass Spec. (FD⁺) m/e 624 (M⁺)

### Example 6

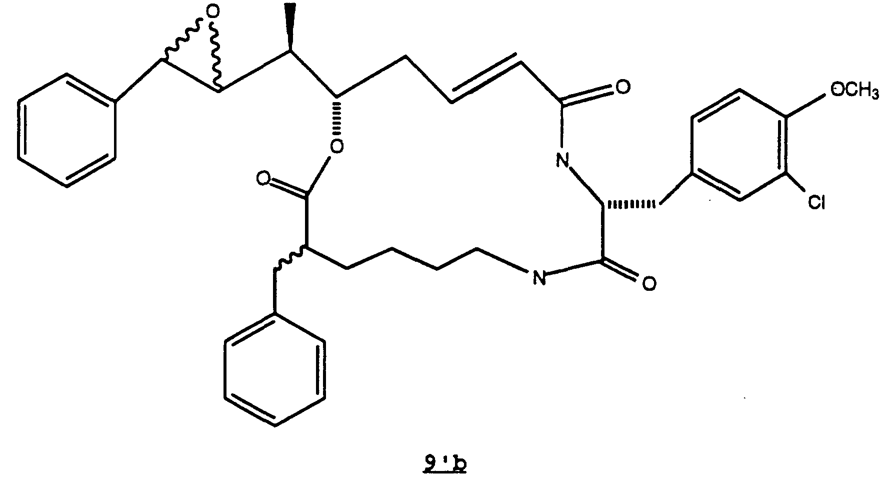

**9'b** was prepared substantially according to the method for the preparation of **9'a**. Mass Spec. (FD⁺ m/e 658. (M⁺).

### Example 7

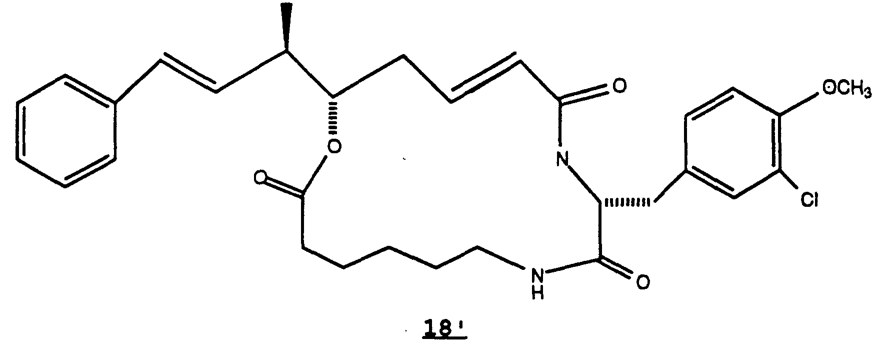

To a solution of 97 mg (0.121 mmol) of **17'** (Preparation 19) in 5 mL CH₂Cl₂ was added 2 mL of trifluoroacetic acid (TFA). The reaction was strirred at 25°C for 30 min, after which it was poured into 25 mL of a saturated aqueous sodium bicarbonate solution. An additional 25 mL of CH₂Cl₂ was added and the organic phase was washed with 1N NaOH, dried over sodium sulfate and concentrated *in vacuo.* This crude material was dissolved in 10 mL of toluene and stirred at 25°C as 58 mg (0.605 mmol) of 2-hydroxypyridine was added. The reaction was stirred at 25°C for 18 h. The reaction was diluted with 50 mL of ethyl acetate and the organic phase washed twice with 1N HCl, twice with brine and twice with saturated sodium bicarbonate solution. The organic solution was dried over sodium sulfate and concentrated *in vacuo*. The crude material was purified by flash chromatography on silica gel using 50% ethyl acetate-hexane as the eluent. The major fraction was concentrated *in vacuo* to give 28 mg (43%) of a white amorphous solid, which was characterized as **18'**. Mass Spec. (FD⁺) m/e 552 (M⁺). HPLC (Reverse Phase, C18, 70% acetonitrile-water, isocratic, 1.0 ml/min.). Retention Times: 6.35 min.

### Example 8

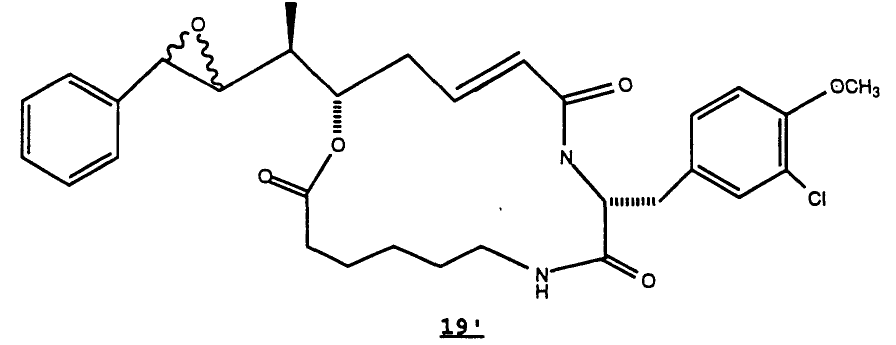

To a solution containing 23 mg (0.043 mmol) of **18'** (Example 7) in 5 mL of CH₂Cl₂ was added 20.8 mg (0.103 mmol) of 85% *m*-chloroperbenzoic acid (mCPBA). The reaction was strirred at 25°C and monitored by HPLC (reverse phase, C₁₈, 70% acetonitrile-water). After about 15 h, the reaction was complete. The reaction was diluted with 25 mL CH₂Cl₂ and washed twice with a saturated sodium meta-bisulfite solution and twice with a saturated sodium bicarbonate solution. The organic layer was dried and concentrated *in vacuo* to give 15 mg (62%) of a white amorphous solid, which was charaterized as a 2:1 mixture of diastereomeric epoxides (ration determined from NMR) of **19'**. HPLC (Reverse Phase, C18, 70% acetonitrile-water, isocratic, 1.0 ml/min.). Retention Time: 4.82 min. (isomers were coincident).

## Claims

1. A compound of Formula I 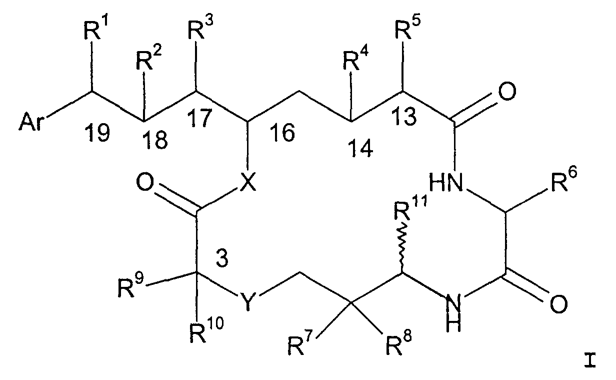 wherein
Ar is phenyl or phenyl substituted with a single group selected from the group consisting of halogen and alkyl of from one to five carbons;
R¹ is halogen, SH, amino, mono(C₁-C₃)alkylamino, di(C₁-C₃)alkylamino, tri (C₁-C₃)alkylammonium, sulfate, or phosphate;
R² is OH or SH; or
R¹ and R² may be taken together to form an-epoxide ring, an aziridine ring, an episulfide ring, a sulfate ring, or a five membered ring consisting of a carbon-carbon-oxygen-phosphorous-oxygen backbone with two additional oxygen atoms, one of which bears an alkyl group of from one to five carbons, connected to the phosphorous atom; or
R¹ and R² may be taken together to form a second bond between C₁₈ and C₁₉;
R³ is an alkyl group of from one to five carbons;
R⁴ is H;
R⁵ is H;
R⁴ and R⁵ may be taken together to form a second bond between C₁₃ and C₁₄;
R⁶ is benzyl, hydroxybenzyl, halohydroxybenzyl, dihalohydroxybenzyl, or substituted benzyl wherein one substituent is a halogen atom and one substituent is an OR¹² group wherein R¹² is alkyl of from one to five atoms;
R⁷ is H or an alkyl group of from one to five carbons;
R⁸ is H or an alkyl group of from one to five carbons;
or R⁷ and R⁸ may optionally be taken together to form a cyclopropyl ring;
R⁹ is selected from the group consisting of H, an alkyl group of one to five carbons, (C₁-C₃) alkylaryl, and aryl, in which aryl means phenyl or naphthyl;
R¹⁰ is selected from the group consisting H, an alkyl group of one to five carbons, (C₁-C₃) alkylaryl, and aryl, in which aryl means phenyl or naphthyl;
R¹¹ is C₁-C₇ alkyl wherein the alkyl may be saturated, unsaturated, branched or straight chain, phenyl, a phenyl group with one to three substituents which may be independently selected from C₁-C₇ alkyl wherein the alkyl
may be saturated, unsaturated, branched or straight chain, Cl, Br, F, and I, benzyl, and a benzyl group with one to three substituents which may be independently selected from C₁-C₇ alkyl wherein the alkyl may be saturated, unsaturated, branched or straight chain, Cl, Br, F, and I;
X is O, NH or C₁-C₃alkylamino;
Y is C, O, NH, S, SO, SO₂ or C₁-C₃alkylamino; or
a pharmaceutically acceptable salt or solvate thereof.

2. A compound as claimed by **Claim 1** wherein Y is O.

3. A compound as claimed by any one of **Claims 1 to 2** wherein X is O.

4. A compound as claimed by any one of **Claims 1 to 3** wherein R⁶ is a group of the formula:

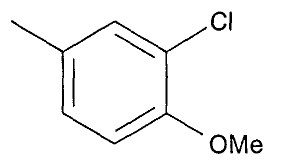

5. A compound as claimed by any one of **Claims 1 to 4** wherein R⁸ and R⁷ are each methyl.

6. A compound as claimed by any one of **Claims 1 to 5** wherein R⁹ is isobutyl and R¹⁰ is hydrogen.

7. A compound as claimed by any one of **Claims 1 to 6** wherein R¹¹ is C₁-C₄ alkyl.

8. A compound as claimed by any one of **Claims 1 to 6** wherein R¹¹ is phenyl.

9. A compound as claimed by any one of **Claims 1 to 8** wherein R¹ and R² form an epoxide group.

10. A compound as claimed by any one of **Claims 1 to 9** wherein Y is C.

11. A compound as claimed by any one of **Claims 1 to 11**, or a pharmaceutically acceptable salt thereof, for use as a pharmaceutical.

12. A compound as claimed by any one of **Claims 1 to 11**, or a pharmaceutically acceptable salt thereof, for use in disrupting a microtubule.

13. A compound as claimed by any one of **Claims 1 to 11**, or a pharmaceutically acceptable salt thereof, for use in treating a neoplasm.

14. A pharmaceutical formulation comprising as active ingredient a compound as claimed by any one of Claims 1 to 11, or a pharmaceutically acceptable salt thereof, associated with one or more excipients or carriers therefor.

15. A compound of Formula III 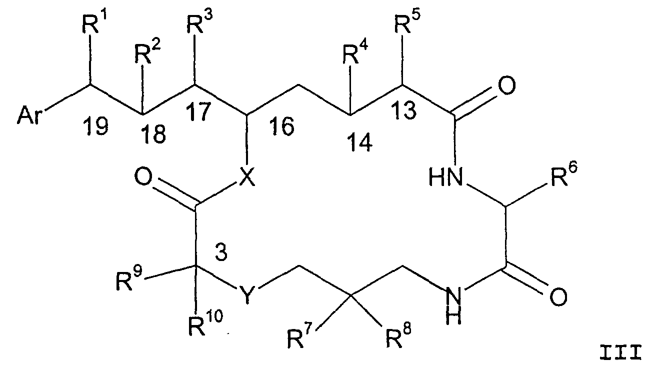 wherein
Ar is phenyl or phenyl substituted with a single group selected from the group consisting of halogen and alkyl of from one to five carbons;
R¹ is halogen, SH, amino, mono(C₁-C₃)alkylamino, di(C₁-C₃)alkylamino, tri (C₁-C₃)alkylammonium, sulfate, or phosphate;
R² is OH or SH; or
R¹ and R² may be taken together with C₁₈ and C₁₉ to form an epoxide ring, an aziridine ring, an episulfide ring, a sulfate ring, or or a five membered ring consisting of a carbon-carbon-oxygen-phosphorous-oxygen backbone with two additional oxygen atoms, one of which bears an alkyl group of from one to five carbons, connected to the phosphorous atom; or
R¹ and R² may be taken together to form a second bond between C₁₈ and C₁₉;
R³ is an alkyl group of from one to five carbons;
R⁴ is H;
R⁵ is H;
R⁴ and R⁵ may be taken together to form a second bond between C₁₃ and C₁₄;
R⁶ is benzyl, hydroxybenzyl, halohydroxybenzyl, dihalohydroxybenzyl, or substituted benzyl wherein one substituent is a halogen atom and one substituent is an OR¹² group wherein R¹² is alkyl of from one to five atoms;
R⁷ is H or an alkyl group of from one to five carbons;
R⁸ is H or an alkyl group of from one to five carbons;
or R⁷ and R⁸ may optionally be taken together to form a cyclopropyl ring;
R⁹ is selected from the group consisting of H, an alkyl group of from one to five carbons, (C₁-C₃) alkylaryl, and aryl, in which aryl means phenyl or naphthyl;
R¹⁰ is selected from the group consisting H, an alkyl group of from one to five carbons, (C₁-C₃) alkylaryl, and aryl, in which aryl means phenyl or naphthyl;
X is O, NH or C₁-C₃ alkylamino;
Y is C, O, NH, S, SO, SO₂ or C₁-C₃ alkylamino; or
a pharmaceutically acceptable salt or solvate thereof.

16. A compound of **Claim 15** wherein Y is O.

17. A compound as claimed by any one of **Claims 15 to 16** wherein X is O.

18. A compound as claimed by any one of **Claims 15 to 17** wherein R⁶ is a group of the formula:

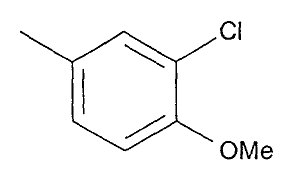

19. A compound as claimed by any one of **Claims 15 to 18** wherein R⁸ and R⁷ are each methyl.

20. A compound as claimed by any one of **Claims 15 to 19** wherein R⁹ is isobutyl and R¹⁰ is hydrogen.

21. A compound as claimed by any one of **Claims 15 to 20** wherein R¹ and R² form an epoxide group.

22. A compound as claimed by any one of **Claims 15 to 21** wherein Y is C.

23. A compound as claimed by any one of **Claims 15 to 22**, or a pharmaceutically acceptable salt thereof, 1 for use as a pharmaceutical.

24. A compound as claimed by any one of **Claims 15 to 22**, or a pharmaceutically acceptable salt thereof, for use in disrupting a microtubule.

25. A compound as claimed by any one of **Claims 15 to 22**, or a pharmaceutically acceptable salt thereof, for use in treating a neoplasm.

26. A pharmaceutical formulation comprising as active ingredient a compound as claimed by any one of **Claims 15 to 22**, or a pharmaceutically acceptable salt thereof, associated with one or more excipients or carriers therefor.

27. A compound selected from the group consisting of 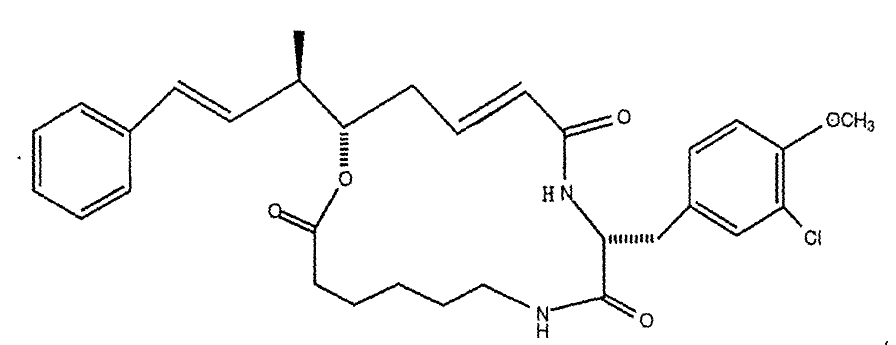 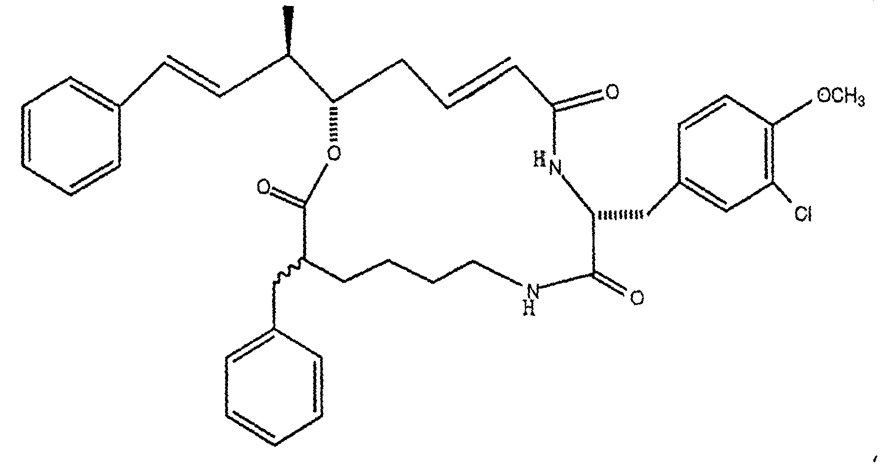 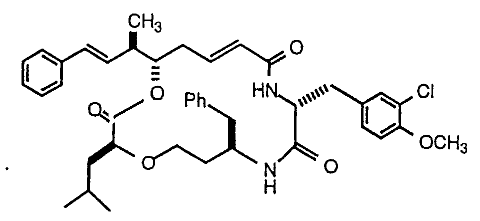 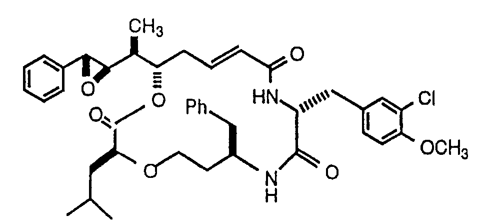 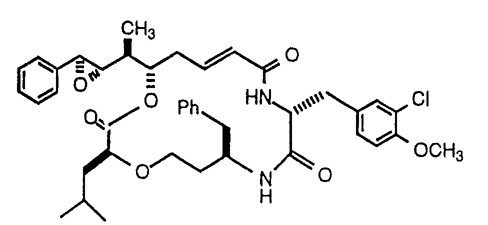 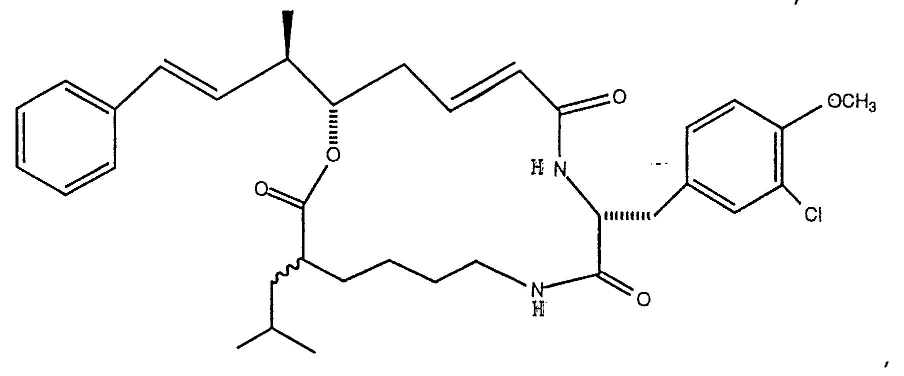 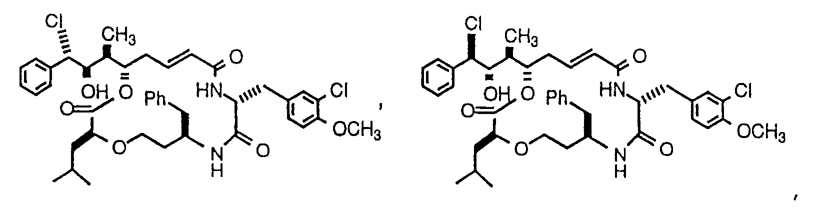 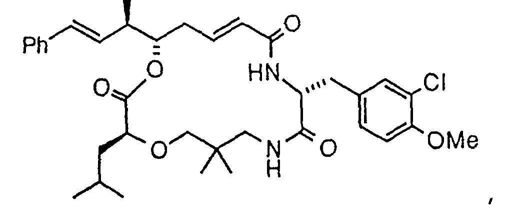 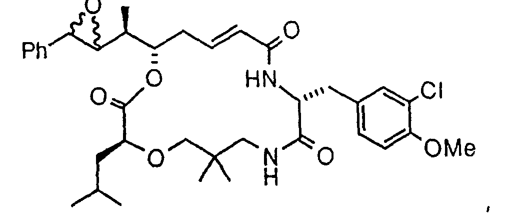 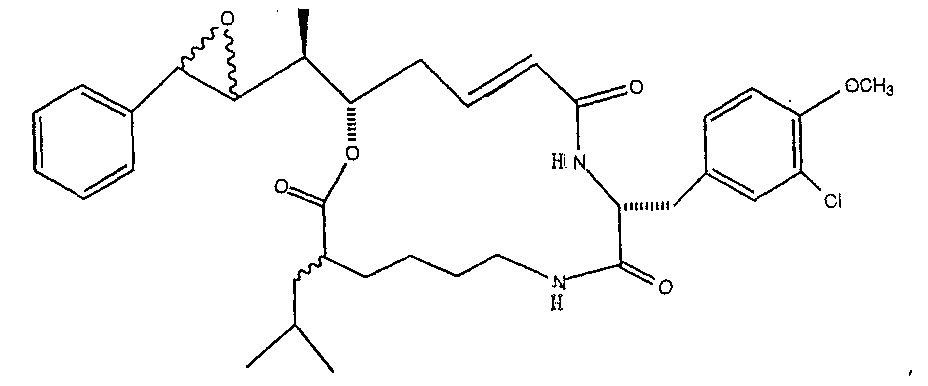 and

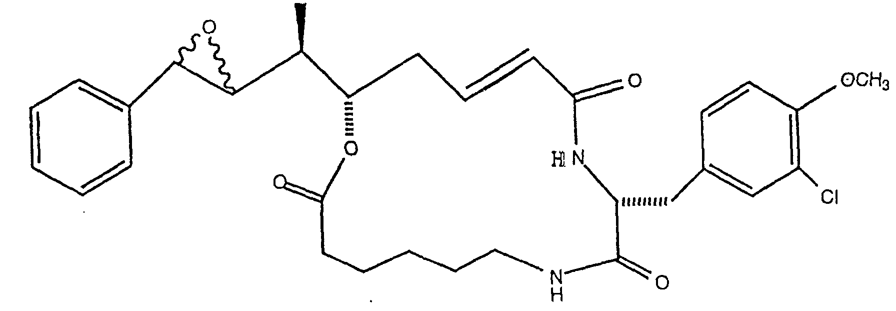

## Patentansprüche

1. Verbindung der Formel I 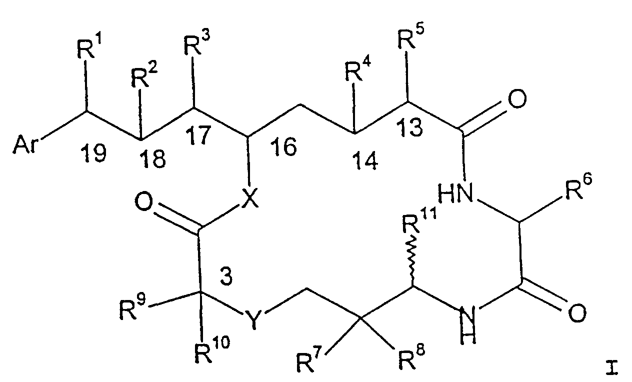 worin
Ar für Phenyl oder Phenyl steht, das mit einer einzelnen Gruppe substituiert ist, ausgewählt aus der Gruppe, die besteht aus Halogen und Alkyl mit einem bis fünf Kohlenstoffatomen,
R¹ für Halogen, SH, Amino, Mono(C₁-C₃)alkylamino, Di(C₁-C₃)alkylamino, Tri(C₁-C₃)alkylammonium, Sulfat oder Phosphat steht,
R² für OH oder SH steht, oder
R¹ und R² unter Bildung eines Epoxidrings, eines Aziridinrings, eines Episulfidrings, eines Sulfatrings oder eines fünfgliedrigen Rings zusammengenommen werden können, der aus einem Kohlenstoff-Kohlenstoff-Sauerstoff-Phosphor-Sauerstoffrückgrad mit zwei zusätzlichen Sauerstoffatomen besteht, von denen eines eine Alkylgruppe mit ein bis fünf Kohlenstoffatomen trägt und mit dem Phosphoratom verbunden ist, oder
R¹ und R² unter Bildung einer zweiten Bindung zwischen C₁₈ und C₁₉ zusammengenommen werden können,
R³ für eine Alkylgruppe mit ein bis fünf Kohlenstoffen steht,
R⁴ für H steht,
R⁵ für H steht,
R⁴ und R⁵ unter Bildung einer zweiten Bindung zwischen C₁₃ und C₁₄ zusammengenommen werden können,
R⁶ für Benzyl, Hydroxybenzyl, Halogenhydroxybenzyl, Dihalogenhydroxybenzyl oder substituiertes Benzyl steht, worin ein Substituent ein Halogenatom ist und ein anderer Substituent eine OR¹² Gruppe ist, worin R¹² für Alkyl mit ein bis fünf Kohlenstoffatomen steht,
R⁷ für H oder eine Alkylgruppe mit ein bis fünf Kohlenstoffatomen steht,
R⁸ für H oder eine Alkylgruppe mit ein bis fünf Kohlenstoffatomen steht, oder
R⁷ und R⁸ wahlweise unter Bildung eines Cyclopropylrings zusammengenommen werden können,
R⁹ aus der Gruppe ausgewählt ist, die besteht aus H, einer Alkylgruppe mit ein bis fünf Kohlenstoffen, (C₁-C₃) Alkylaryl und Aryl, worin Aryl für Phenyl oder Naphthyl steht,
R¹⁰ aus der Gruppe ausgewählt ist, die besteht aus H, einer Alkylgruppe mit ein bis fünf Kohlenstoffatomen, (C₁-C₃) Alkylaryl und Aryl, worin Aryl für Phenyl oder Naphthyl steht,
R¹¹ steht für C₁-C₇ Alkyl, worin Alkyl gesättigt, ungesättigt, verzweigt oder geradkettig sein kann, Phenyl eine Phenylgruppe mit ein bis drei Substituenten, die unabhängig ausgewählt werden aus C₁-C₇ Alkyl, worin das Alkyl gesättigt, ungesättigt, verzweigt oder geradkettig sein kann, Cl, Br, F und I, Benzyl und Benzylgruppe mit ein bis drei Substituenten, die unabhängig ausgewählt sind aus C₁-C₇ Alkyl, worin das Alkyl gesättigt, ungesättigt, verzweigt oder geradkettig sein kann, Cl, Br, F und I,
X für O, NH oder C₁-C₃ Alkylamino steht,
Y für C, O, NH, S, SO, SO₂ oder C₁-C₃ Alkylamino steht,
oder ein pharmazeutisch annehmbares Salz oder Solvat hiervon.

2. Verbindung nach Anspruch 1, worin Y für O steht.

3. Verbindung nach einem der Ansprüche 1 bis 2, worin X für O steht.

4. Verbindung nach einem der Ansprüche 1 bis 3, worin R⁶ für eine Gruppe der folgenden Formel steht

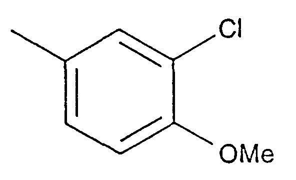

5. Verbindung nach einem der Ansprüche 1 bis 4, worin R⁸ und R⁷ jeweils für Methyl stehen.

6. Verbindung nach einem der Ansprüche 1 bis 5, worin R⁹ für Isobutyl und R¹⁰ für Wasserstoff steht.

7. Verbindung nach einem der Ansprüche 1 bis 6, worin R¹¹ für C₁-C₄ Alkyl steht.

8. Verbindung nach einem der Ansprüche 1 bis 6, worin R¹¹ für Phenyl steht.

9. Verbindung nach einem der Ansprüche 1 bis 8, worin R¹ und R² eine Epoxidgruppe bilden.

10. Verbindung nach einem der Ansprüche 1 bis 9, worin Y für C steht.

11. Verbindung nach einem der Ansprüche 1 bis 10 oder ein pharmazeutisch annehmbares Salz hiervon zur Verwendung als Pharmazeutikum.

12. Verbindung nach einem der Ansprüche 1 bis 11 oder ein pharmazeutisch annehmbares Salz hiervon zur Verwendung bei der Zerstörung eines Mikrotubulus.

13. Verbindung nach einem der Ansprüche 1 bis 11 oder ein pharmazeutisch annehmbares Salz hiervon zur Behandlung eines Neoplasmas.

14. Pharmazeutische Formulierung, die als Wirkstoff eine Verbindung nach einem der Ansprüche 1 bis 11 oder ein pharmazeutisch annehmbares Salz hiervon zusammen mit einem oder mehreren Hilfsstoffen oder Trägern hierfür enthält.

15. Verbindung der Formel III 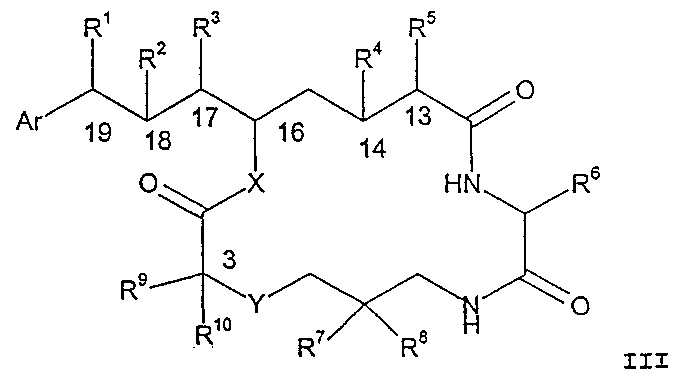 worin
Ar für Phenyl oder Phenyl steht, das mit einer einzelnen Gruppe substituiert ist, ausgewählt aus der Gruppe, die besteht aus Halogen und Alkyl mit einem bis fünf Kohlenstoffatomen,
R¹ für Halogen, SH, Amino, Mono(C₁-C₃)alkylamino, Di(C₁-C₃)alkylamino, Tri(C₁-C₃)alkylammonium, Sulfat oder Phosphat steht,
R² für OH oder SH steht, oder
R¹ und R² mit C₁₈ und C₁₉ unter Bildung eines Epoxidrings, eines Aziridinrings, eines Episulfidrings, eines Sulfatrings oder eines fünfgliedrigen Rings zusammengenommen werden können, der aus einem Kohlenstoff-Kohlenstoff-Sauerstoff-Phosphor-Sauerstoffrückgrad mit zwei zusätzlichen Sauerstoffatomen besteht, von denen eines eine Alkylgruppe mit ein bis fünf Kohlenstoffatomen trägt und mit dem Phosphoratom verbunden ist, oder
R¹ und R² unter Bildung einer zweiten Bindung zwischen C₁₈ und C₁₉ zusammengenommen werden können,
R³ für eine Alkylgruppe mit ein bis fünf Kohlenstoffen steht,
R⁴ für H steht,
R⁵ für H steht,
R⁴ und R⁵ unter Bildung einer zweiten Bindung zwischen C₁₃ und C₁₄ zusammengenommen werden können,
R⁶ für Benzyl, Hydroxybenzyl, Halogenhydroxybenzyl, Dihalogenhydroxybenzyl oder substituiertes Benzyl steht, worin ein Substituent ein Halogenatom ist und ein anderer Substituent eine OR¹² Gruppe ist, worin R¹² für Alkyl mit ein bis fünf Kohlenstoffatomen steht,
R⁷ für H oder eine Alkylgruppe mit ein bis fünf Kohlenstoffatomen steht,
R⁸ für H oder eine Alkylgruppe mit ein bis fünf Kohlenstoffatomen steht, oder
R⁷ und R⁸ wahlweise unter Bildung eines Cyclopropylrings zusammengenommen werden können,
R⁹ aus der Gruppe ausgewählt ist, die besteht aus H, einer Alkylgruppe mit ein bis fünf Kohlenstoffen, (C₁-C₃)Alkylaryl und Aryl, worin Aryl für Phenyl oder Naphthyl steht,
R¹⁰ aus der Gruppe ausgewählt ist, die besteht aus H, einer Alkylgruppe mit ein bis fünf Kohlenstoffatomen, (C₁-C₃) Alkylaryl und Aryl, worin Aryl für Phenyl oder Naphthyl steht,
X für O, NH oder C₁-C₃ Alkylamino steht,
Y für C, O, NH, S, SO, SO₂ oder C₁-C₃ Alkylamino steht,
oder ein pharmazeutisch annehmbares Salz oder Solvat hiervon.

16. Verbindung nach Anspruch 15, worin Y für O steht.

17. Verbindung nach einem der Ansprüche 15 und 16, worin X für O steht.

18. Verbindung nach einem der Ansprüche 15 bis 17, worin R⁶ für eine Gruppe der folgenden Formel steht:

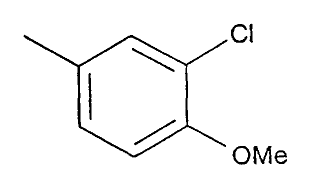

19. Verbindung nach einem der Ansprüche 15 bis 18, worin R⁸ und R⁷ jeweils für Methyl stehen.

20. Verbindung nach einem der Ansprüche 15 bis 19, worin R⁹ für Isobutyl und R¹ für Wasserstoff steht.

21. Verbindung nach einem der Ansprüche 15 bis 20, worin R¹ und R² eine Epoxidgruppe bilden.

22. Verbindung nach einem der Ansprüche 15 bis 21, worin Y für C steht.

23. Verbindung nach einem der Ansprüche 15 bis 22 oder ein pharmazeutisch annehmbares Salz hiervon zur Verwendung als Pharmazeutikum.

24. Verbindung nach einem der Ansprüche 15 bis 22 oder ein pharmazeutisch annehmbares Salz hiervon zur Verwendung bei der Zerstörung eines Mikrotubulus.

25. Verbindung nach einem der Ansprüche 15 bis 22 oder ein pharmazeutisch annehmbares Salz hiervon zur Behandlung eines Neoplasmas.

26. Pharmazeutische Formulierung, die als Wirkstoff eine Verbindung nach einem der Ansprüche 15 bis 22 oder ein pharmazeutisch annehmbares Salz hiervon zusammen mit einem oder mehreren Hilfsstoffen oder Trägern hierfür enthält.

27. Verbindung ausgewählt aus der Gruppe, dir besteht aus 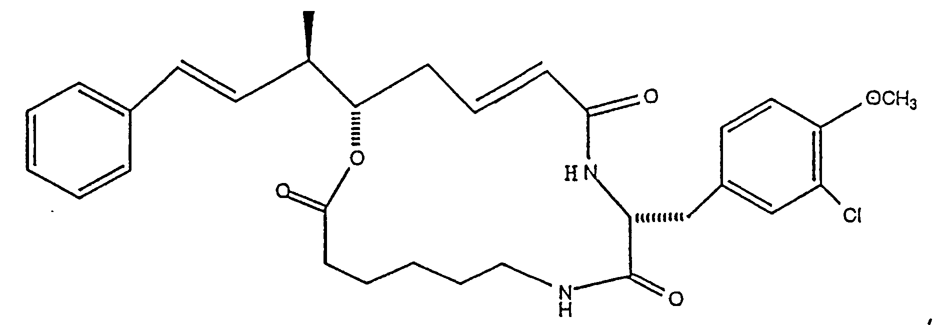 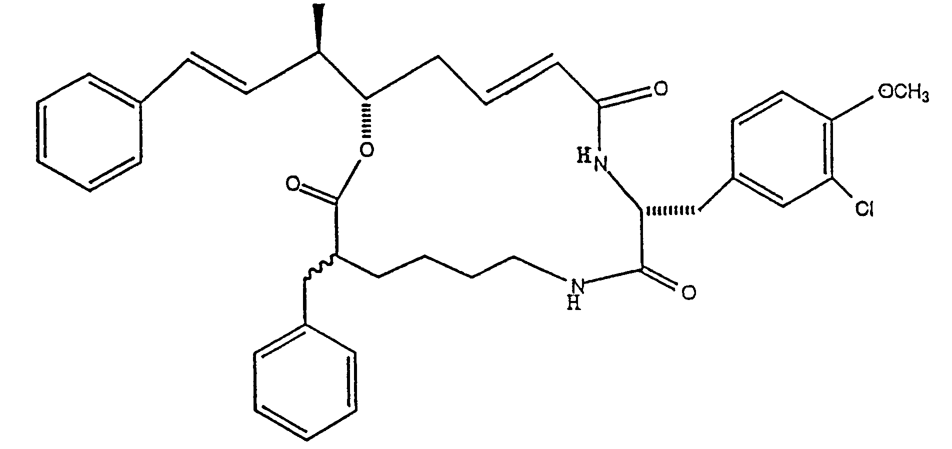 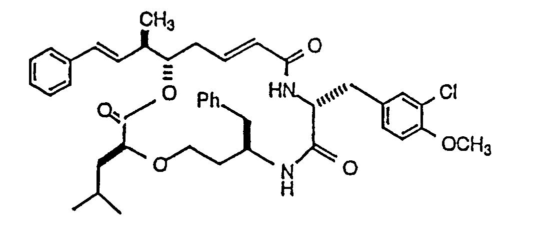 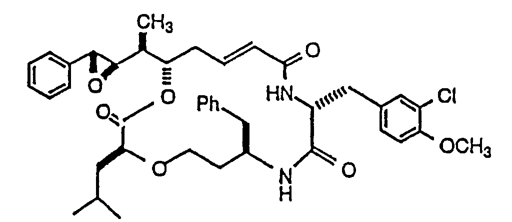 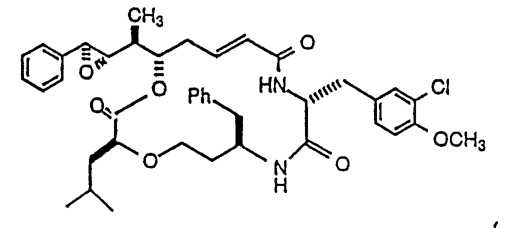 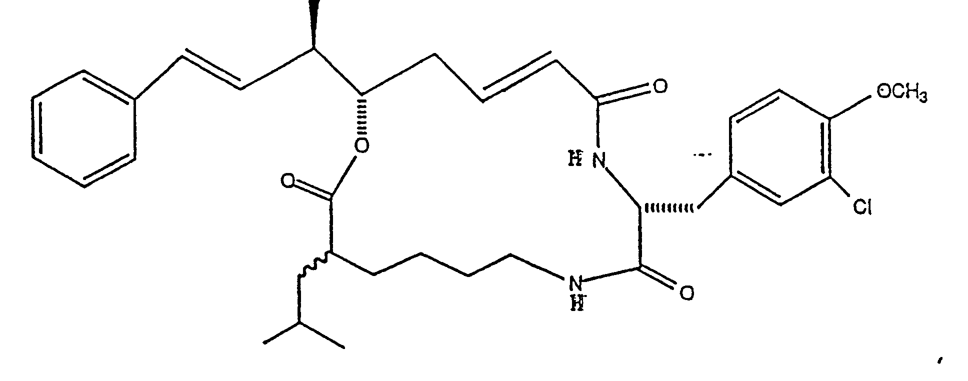 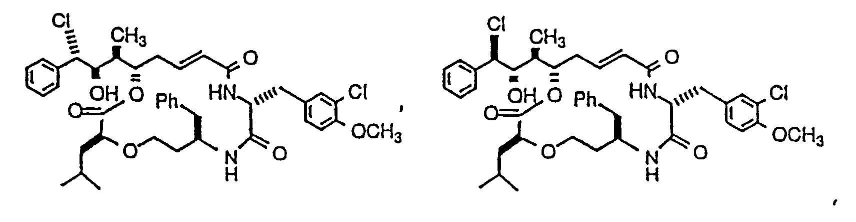 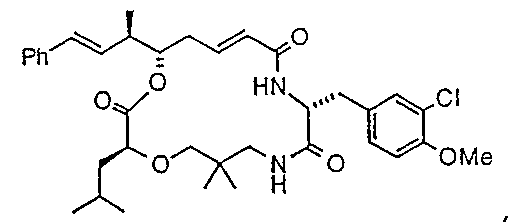 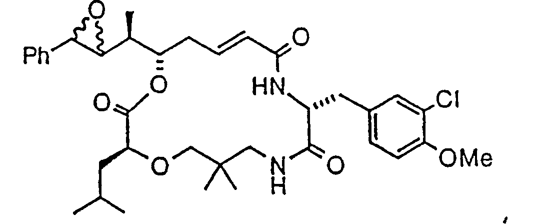 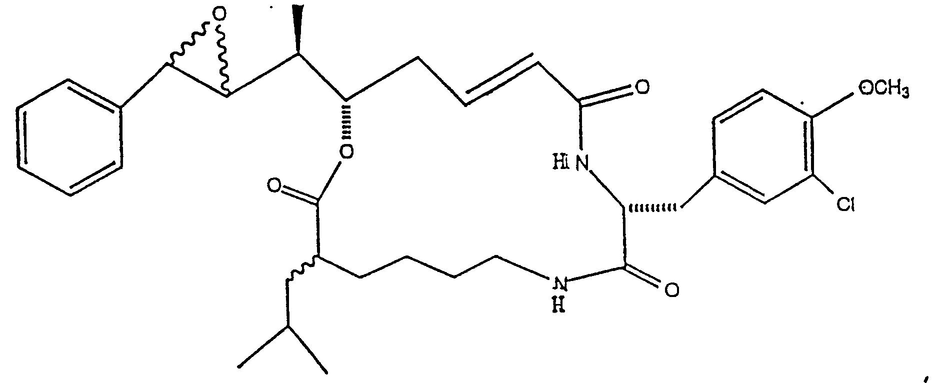 und

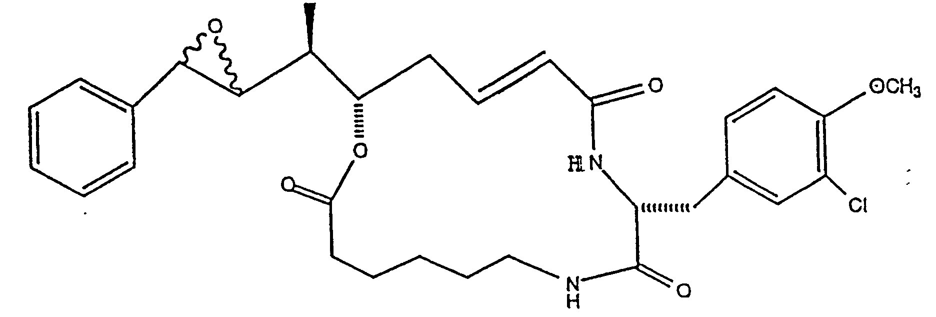

## Revendications

1. Composé répondant à la formule I 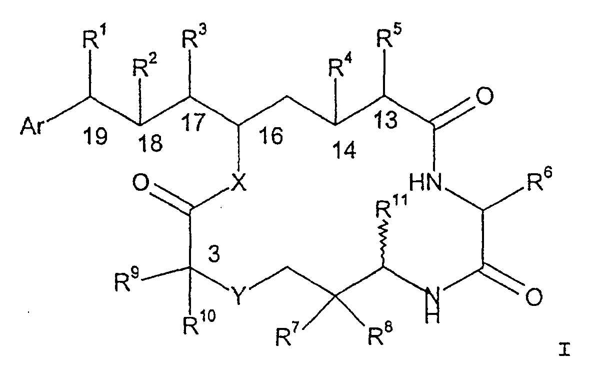 dans laquelle
Ar représente un groupe phényle ou un groupe phényle substitué avec un groupe unique choisi parmi le groupe constitué par un atome d'halogène et par un groupe alkyle contenant de un à cinq atomes de carbone;
R¹ représente un atome d'halogène, un groupe SH, un groupe amino, un groupe monoalkyl(en C₁-C₃)amino, un groupe dialkyl(en C₁-C₃)amino, un groupe trialkyl(en C₁-C₃)ammonium, un groupe sulfate ou un groupe phosphate;
R² représente un groupe OH ou un groupe SH; ou bien
R¹ et R² peuvent être pris ensemble pour former un noyau époxyde, un noyau aziridine, un noyau épisulfure, un noyau sulfate ou un noyau à cinq membres constitué d'un squelette de carbone-carbone-oxygène-phosphore-oxygène comprenant deux atomes d'oxygène supplémentaires, dont un porte un groupe alkyle contenant de un à cinq atomes de carbone, liés à l'atome de phosphore; ou bien
R¹ et R² peuvent être pris ensemble pour former une seconde liaison entre C₁₈ et C₁₉;
R³ représente un groupe alkyle contenant de un à cinq atomes de carbone;
R⁴ représente un atome d'hydrogène;
R⁵ représente un atome d'hydrogène;
R⁴ et R⁵ peuvent être pris ensemble pour former une seconde liaison entre C₁₃ et C₁₄;
R⁶ représente un groupe benzyle, un groupe hydroxybenzyle, un groupe halogénohydroxybenzyle, un groupe dihalogénohydroxybenzyle ou un groupe benzyle substitué dans lequel un substituant représente un atome d'halogène et un substituant représente un groupe OR¹² où R¹² représente un groupe alkyle contenant de un à cinq atomes de carbone;
R⁷ représente un atome d'hydrogène ou un groupe alkyle contenant de un à cinq atomes de carbone;
R⁸ représente un atome d'hydrogène ou un groupe alkyle contenant de un à cinq atomes de carbone;
ou bien R⁷ et R⁸ peuvent, le cas échéant, être pris ensemble pour former un noyau cyclopropyle;
R⁹ est choisi parmi le groupe constitué par un atome d'hydrogène, un groupe alkyle contenant de un à cinq atomes de carbone, un groupe alkylaryle en C₁-C₃ et un groupe aryle, le groupe aryle représentant un groupe phényle ou un groupe naphtyle;
R¹⁰ est choisi parmi le groupe constitué par un atome d'hydrogène, un groupe alkyle contenant de un à cinq atomes de carbone, un groupe alkylaryle en C₁-C₃ et un groupe aryle, le groupe aryle représentant un groupe phényle ou un groupe naphtyle;
R¹¹ représente un groupe alkyle en C₁-C₇ dans lequel le groupe alkyle peut être saturé, non saturé, à chaîne droite ou ramifiée, un groupe phényle, un groupe phényle comprenant de un à trois substituants qui peuvent être choisis indépendamment l'un de l'autre parmi le groupe contenant un groupe alkyle en C₁-C₇ dans lequel le groupe alkyle peut être saturé, non saturé, à chaîne droite ou ramifiée, un atome de chlore, un atome de brome, un atome de fluor et un atome d'iode, un groupe benzyle et un groupe benzyle contenant de un à trois substituants qui peuvent être choisis indépendamment l'un de l'autre parmi le groupe comprenant un groupe alkyle en C₁-C₇ dans lequel le groupe alkyle peut être saturé, non saturé, à chaîne droite ou ramifiée, un atome de chlore, un atome de brome, un atome de fluor et un atome d'iode;
X représente un atome d'oxygène, un groupe NH ou un groupe alkyl(en C₁-C₃)amino;
Y représente un atome de carbone, un atome d'oxygène, un groupe NH, un atome de soufre, un groupe SO, un groupe SO₂ ou un groupe alkyl(en C₁-C₃)amino; ou
un de ses sels ou de ses solvates pharmaceutiquement acceptables.

2. Composé selon la revendication 1, dans lequel Y représente un atome d'oxygène.

3. Composé selon l'une quelconque des revendications 1 à 2, dans lequel X représente un atome d'oxygène.

4. Composé selon l'une quelconque des revendications 1 à 3, dans lequel R⁶ représente un groupe répondant à la formule:

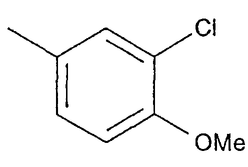

5. Composé selon l'une quelconque des revendications 1 à 4, dans lequel R⁸ et R⁷ représentent chacun un groupe méthyle.

6. Composé selon l'une quelconque des revendications 1 à 5, dans lequel R⁹ représente un groupe isobutyle et R¹⁰ représente un atome d'hydrogène.

7. Composé selon l'une quelconque des revendications 1 à 6, dans lequel R¹¹ représente un groupe alkyle en C₁-C₄.

8. Composé selon l'une quelconque des revendications 1 à 6, dans lequel R¹¹ représente un groupe phényle.

9. Composé selon l'une quelconque des revendications 1 à 8, dans lequel R¹ et R² forment un groupe époxyde.

10. Composé selon l'une quelconque des revendications 1 à 9, dans lequel Y représente un atome de carbone.

11. Composé selon l'une quelconque des revendications 1 à 10 ou un de ses sels pharmaceutiquement acceptables à utiliser comme produit pharmaceutique.

12. Composé selon l'une quelconque des revendications 1 à 11 ou un de ses sels pharmaceutiquement acceptables à utiliser pour rompre des microtubules.

13. Composé selon l'une quelconque des revendications 1 à 11 ou un de ses sels pharmaceutiquement acceptables à utiliser pour traiter des néoplasmes.

14. Formulation pharmaceutique comprenant, à titre d'ingrédient actif, un composé selon l'une quelconque des revendications 1 à 11, ou un de ses sels pharmaceutiquement acceptables, en association avec un ou plusieurs excipients ou supports pour le composé.

15. Composé répondant à la formule III 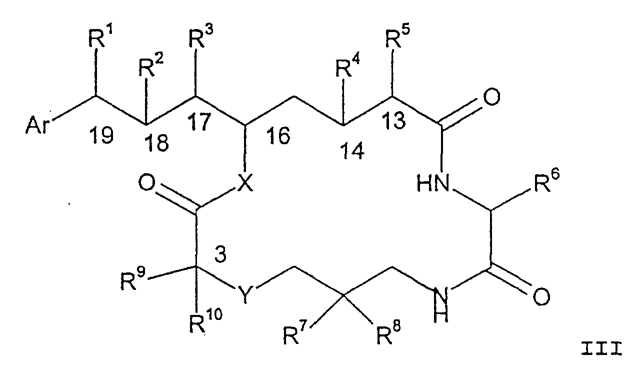 dans laquelle
Ar représente un groupe phényle ou un groupe phényle substitué avec un groupe unique choisi parmi le groupe constitué par un atome d'halogène et par un groupe alkyle contenant de un à cinq atomes de carbone;
R¹ représente un atome d'halogène, un groupe SH, un groupe amino, un groupe monoalkyl(en C₁-C₃)amino, un groupe dialkyl(en C₁-C₃)amino, un groupe trialkyl(en C₁-C₃)ammonium, un groupe sulfate ou un groupe phosphate;
R² représente un groupe OH ou un groupe SH; ou bien
R¹ et R² peuvent être pris ensemble avec C₁₈ et C₁₉ pour former un noyau époxyde, un noyau aziridine, un noyau épisulfure, un noyau sulfate ou un noyau à cinq membres constitué d'un squelette de carbone-carbone-oxygène-phosphore-oxygène comprenant deux atomes d'oxygène supplémentaires, dont un porte un groupe alkyle contenant de un à cinq atomes de carbone, liés à l'atome de phosphore; ou bien
R¹ et R² peuvent être pris ensemble pour former une seconde liaison entre C₁₈ et C₁₉;
R³ représente un groupe alkyle contenant de un à cinq atomes de carbone;
R⁴ représente un atome d'hydrogène;
R⁵ représente un atome d'hydrogène;
R⁴ et R⁵ peuvent être pris ensemble pour former une seconde liaison entre C₁₃ et C₁₄;
R⁶ représente un groupe benzyle, un groupe hydroxybenzyle, un groupe halogénohydroxybenzyle, un groupe dihalogénohydroxybenzyle ou un groupe benzyle substitué dans lequel un substituant représente un atome d'halogène et un substituant représente un groupe OR¹² où R¹² représente un groupe alkyle contenant de un à cinq atomes de carbone;
R⁷ représente un atome d'hydrogène ou un groupe alkyle contenant de un à cinq atomes de carbone;
R⁸ représente un atome d'hydrogène ou un groupe alkyle contenant de un à cinq atomes de carbone;
ou bien R⁷ et R⁸ peuvent, le cas échéant, être pris ensemble pour former un noyau cyclopropyle;
R⁹ est choisi parmi le groupe constitué par un atome d'hydrogène, un groupe alkyle contenant de un à cinq atomes de carbone, un groupe alkylaryle en C₁-C₃ et un groupe aryle, le groupe aryle représentant un groupe phényle ou un groupe naphtyle;
R¹⁰ est choisi parmi le groupe constitué par un atome d'hydrogène, un groupe alkyle contenant de un à cinq atomes de carbone, un groupe alkylaryle en C₁-C₃ et un groupe aryle, le groupe aryle représentant un groupe phényle ou un groupe naphtyle;
X représente un atome d'oxygène, un groupe NH ou un groupe alkyl(en C₁-C₃)amino;
Y représente un atome de carbone, un atome d'oxygène, un groupe NH, un atome de soufre, un groupe SO, un groupe SO₂ ou un groupe alkyl(en C₁-C₃)amino; ou
un de ses sels ou de ses solvates pharmaceutiquement acceptables.

16. Composé selon la revendication 15, dans lequel Y représente un atome d'oxygène.

17. Composé selon l'une quelconque des revendications 15 à 16, dans lequel X représente un atome d'oxygène.

18. Composé selon l'une quelconque des revendications 15 à 17, dans lequel R⁶ représente un groupe répondant à la formule:

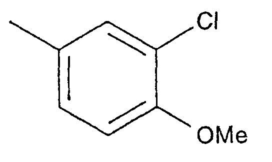

19. Composé selon l'une quelconque des revendications 15 à 18, dans lequel R⁸ et R⁷ représentent chacun un groupe méthyle.

20. Composé selon l'une quelconque des revendications 15 à 19, dans lequel R⁹ représente un groupe isobutyle et R¹⁰ représente un atome d'hydrogène.

21. Composé selon l'une quelconque des revendications 15 à 20, dans lequel R¹ et R² forment un groupe époxyde.

22. Composé selon l'une quelconque des revendications 15 à 21, dans lequel Y représente un atome de carbone.

23. Composé selon l'une quelconque des revendications 15 à 22 ou un de ses sels pharmaceutiquement acceptables à utiliser comme produit pharmaceutique.

24. Composé selon l'une quelconque des revendications 15 à 22 ou un de ses sels pharmaceutiquement acceptables à utiliser pour rompre des microtubules.

25. Composé selon l'une quelconque des revendications 15 à 22 ou un de ses sels pharmaceutiquement acceptables à utiliser pour traiter des néoplasmes.

26. Formulation pharmaceutique comprenant, à titre d'ingrédient actif, un composé selon l'une quelconque des revendications 15 à 22, ou un de ses sels pharmaceutiquement acceptables, en association avec un ou plusieurs excipients ou supports pour le composé.

27. Composé choisi parmi le groupe constitué par:
